(19) Europäisches Patentamt
European Patent Office
Office européen des brevets

(11) Publication number: **0 412 158 A1**

(12) **EUROPEAN PATENT APPLICATION published in accordance with Art. 158(3) EPC**


(21) Application number: **88905227.0**

(51) Int. Cl.[5]: **A61K 31/28**, A61K 33/24

(22) Date of filing: **10.06.88**

(86) International application number: **PCT/JP88/00559**

(87) International publication number: **WO 88/09658 (15.12.88 88/27)**

(30) Priority: **11.06.87 JP 144197/87**
**29.07.87 JP 189762/87**
**05.08.87 JP 194316/87**
**30.03.88 JP 74464/88**

(43) Date of publication of application: **13.02.91 Bulletin 91/07**

(84) Designated Contracting States: **BE CH DE FR GB IT LI**



(71) Applicant: **TERUMO KABUSHIKI KAISHA**
**No. 44-1, Hatagaya 2-chome, Shibuya-ku**
**Tokyo 151(JP)**

Applicant: **Yamase, Toshihiro**
**13-905, Wakabadai 4-chome, Asahi-ku**
**Yokohama-shi, Kanagawa-ken(JP)**

Applicant: **Fujita, Haruhisa**
**11-8, Shoudo 2-chome, Sakae-ku**
**Yokohama-shi, Kanagawa-ken(JP)**

Applicant: **Fukushima, Kouji**
**23-5-3, Hijirigaoka 2-chome**
**Tama-shi, Tokyo(JP)**

(72) Inventor: **SETO, Yoshiko**
**2-3-406, Natsumidai 1-chome**
**Funabashi-shi Chiba 273(JP)**

(74) Representative: **Gillard, Marie-Louise et al**
**Cabinet Beau de Loménie 55, Rue d'Amsterdam**
**F-75008 Paris(FR)**


(54) **ONCOSTATIC DRUG.**


(57) An oncostatic drug which contains as the effective ingredient a polyacid salt of molybdenum or a heteropolyacid salt thereof with tungsten represented by the general formulae (I) to (III): $[A]m[MOx\ 0y\ Hz]n.lH_2O$ (I); $[B]p[MOu\ O\ w(O_2)t(0H)v] \bullet KH_2O$ (II);$[D]qHr[XM\ O\ sW6\text{-}sO_{24}]$(III),wherein A represents(α)(wherein $R^1$ to $R^4$ each represents a lower alkyl),an alkali metal, or a mixture of an alkali metal with an other alkali metal, H, $NH_4$ or an alkylammonium, B and D each represents an alkali metal, $NH_4$ or an alkylammonium, X represents I, Cr, Co, Pt, Te, Ni, Mn, Al, Ga, Zn, Fe or Cu, and m, n, p, q, r, s, t, u, v, w, x, y and z each represents O or an integer.

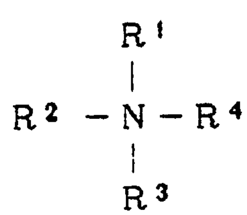

(α)


EP 0 412 158 A1

# ANTINEOPLASTIC DRUG

## Technical Field

This invention relates to an antineoplastic drug. More particularly, this invention relates to an antineoplastic drug comprising a polymolybdate, especially an antineoplastic drug manifesting conspicuous efficacy for solid tumors.

## Background Art

The methods currently available for the treatment of malignant tumors include surgical operation, chemotherapy, immunotherapy, and radiotherapy, for example. In these methods, the surgical operation and the radiotherapy are most significant in terms of radicality of treatment. Since the surgical operation and the radiotherapy are both local treatments, they are effective so long as the tumors under treatment are restrained with the category of local lesions. They exhibit greatly limited efficacy when the tumors are so progressive as to surpass the category of local lesions or they are systemic deseases.

The chemotherapy and the immunotherapy are characteristically intended as systemic treatments and are both methods of treatments and are both methods of treatment in a relatively new field. They, however, are showing a remarkable advance and are anxiously expected to continue growth in the future.

The surgical operation, the radiotherapy, and the chemotherapy are therapeutic methods severally different in principle, and their effects and limits are different automatically. In a proper combination of such therapeutic methods, the component methods synergistically act upon one another or complement one another and serve the purpose of enhancing the overall effect of treatment. The therapy of malignant tumors is improved only by such a multiphasic therapeutic process or a composite therapeutic method as described above. Among these composite therapeutic methods, the chemotherapy has been steadily gaining in practical significance.

In the chemotherapy, the method resorting to combined use of a plurality of agents including Adriamycin has been gaining in growth. The strong secondary effects of such agents, however, restrain the chemotherapy within the category of a narrow supplementary therapy. On account of the secondary effects, the actual application of the chemotherapy is greatly limited quantitatively and the results thereof are not fully satisfactory.

Numerous kinds of antineoplastic drugs have been developed and put to use recently. They have both merits and demerits in terms of antineoplastic effect and toxicity (secondary reaction). None of these antineoplastic drugs has fully satisfied necessary conditions. In the circumstances, an earnest desire is expressed in the industry for the development of a new type of antineoplastic drug.

An object of this invention, therefore, is to provide a novel antineoplastic drug.

Another object of this invention is to provide and antineoplastic drug which possesses a distinct ability to supress the hyperplasia of tumors and entails secondary effects very sparingly.

## Disclosure of Invention

The objects described above are accomplished by an antineoplastic drug having as an active component thereof a polymolybdate represented by the following general formula I:

$$[A]_m\ [Mo_x\ O_y\ H_z]_n \cdot \ell H_2O$$

wherein A stands for

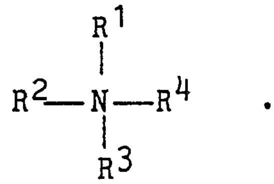

[I]

wherein $R^1$, $R^2$, $R^3$, and R4 independently stand for hydrogen atom or a lower alkyl group, which may be identical or not, an alkali metal atom, or a mixture of an alkali metal atom with other alkali metal atom, hydrogen atom, an ammonium group, or an alkyl ammonium group, providing that the number of carbon atoms of the alkyl group is an integer in the range of 1 to 3, i.e. an alkali metal atom, an alkali metal atom-other alkali metal atom combination, an alkali metal atomp-hydrogen atom combination, an alkali metal atom-ammonium combination, or an alkali metal atom-alkyl ammonium combination, m for an integer in the range of 1 to 16, n for an integer such that the negative charge, xn, assumes the value -m, x for an integer in the range of 2 to 36, y for an integer in the range of 4 to 112, z for an integer in the range of 0 to 24, and $\ell$ for an integer in the range of 0 to 80. This invention further discloses an antineoplastic drug wherein an alkali metal salt of polymolybdic acid is in the form of an aqueous solution. This invention further discloses an antineoplastic drug wherein the aqueous solution is stable at a pH value in the range of 4 to 9, preferably 6 to 8.

Further, this invention discloses an antineoplastic drug in the general formula I of which $R^1$ stands for hydrogen atom, $R^2$, $R^3$, and $R^4$ independently stand for hydrogen atom or an alkyl group of 1 to 6 carbon atoms, except for the case in which $R^2$ to $R^4$ invariably stand for hydrogen atom, m stands for an integer in the range of 2 to 8, n for an integer in the range of 2 to 8, x for an integer in the range of 2 to 20, y for an integer in the range of 10 to 50, z for an integer in the range of 0 to 10, and $\ell$ for an integer in the range of 1 to 10. This invention further discloses an antineoplastic drug which is intended for solid tumors.

This invention further discloses an antineoplastic drug wherein the alkali metal salt of polymolybdic acid is represented by the following general formula:

$Y_4$ $[Mo_7\ O_{24}]$ • $4H_2O$ wherein Y stands for an alkali metal atom or the mixture of an alkali metal atom with other alkali metal atom, hydrogen atom, an ammonium group, or an alkyl ammonium group, providing that the number of carbon atoms of the alkyl group is an integer in the range of 1 to 3. Further, this invention discloses an antineoplastic drug wherein the alkali metal salt of polymolybdic acid is represented by the formula, $K_6$ $[Mo_7\ O_{24}]$ • $4H_2O$. This invention further discloses an antineoplastic drug wherein the alkali metal salt of polymolybdic acid is represented by the formula, $Na_6$ $[Mo_7\ O_{24}]$ • $4H_2O$. This invention further discloses an antineoplastic drug wherein the alkali metal salt of polymolybdic acid is represented by the formula, $Li_6$ $[Mo_7\ O_{24}]$ ++++++ • $4H_2O$. This invention further discloses an antineoplastic drug wherein the alkali metal salt of polymolybdic acid is represented by the formula, $Cs_6$ $[Mo_7\ O_{24}]$ • $4H_2O$. The objects described above are further accomplished by an antineoplastic drug having as a principal component thereof a polymolybdate represented by the following general formula II:

$$B_p[MouOw(O_2)t(OH)v] \cdot KH_2O \qquad (II)$$

wherein B stands for an alkali metal atoms, ammonium group, an alkyl ammonium groups, and a mixture thereof, p for an integer in the range of 2 to 10, u for an integer in the range of 3 to 10, w for an integer in the range of 1 to 30, t for an integer in the range of 1 to 15, v for an integer in the range of 0 to 5, and k for an integer in the range of 0 to 20.

This invention further discloses an antineoplastic drug wherein the polymolybdate is in the form of an aqueous solution. This invention further discloses an antineoplastic drug wherein the aqueous solution is stable at a pH value in the range of 4 to 9. This invention further discloses an antineoplastic drug wherein the polymolybdate is represented by the following general formula: $[B]_6$ $[Mo_7O_{22}(O_2)_2]$ • $KH_2O$

wherein B independently stands for an alkali metal atoms, ammonium group, alkylammonium groups and a mixture thereof, and k for an integer in the range of 0 to 20. This invention further discloses an antineoplastic drug wherein the polymolybdate is represented by the formula, $K_6$ $[Mo_7\ O_{22}\ (O_2)_2]$ • $8H_2O$. This invention further discloses an antineoplastic drug wherein the polymolybdate is represented by the formula, $[NH_3\ (iso)_6]$ $[Mo_7\ O_{22}\ (\ O_2)_2]$ • $5H_2O$ wherein iso stands for isopropyl group. This invention also discloses an antineoplastic drug wherein the polymolybdate is represented by the formula, $[B]_6$ $[Mo_7\ O_{23}$ - $(O_2)]$ • $kH_2O$ wherein B stands an alkali metal atoms, ammonium group, alkylammonium group and a mixture there of, and k for an integer in the range of 0 to 20. This invention further discloses an antineoplastic drug wherein polymolybdate is represented by the formula, $K_6$ $[Mo_7\ O_{23}\ (O_2)]$ • $8H_2O$. This invention further discloses an antineoplastic drug wherein the polymolybdate is represented by the formula, $[NH_3\ (iso)]_6$ $[Mo_7\ O_{23}\ (O_2)]$ • $5H_2O$

wherein iso stands for isopropyl group.

The objects described above are also accomplished by an antineoplastic drug having as a principal component thereof a heteropoly acid salt of molybdenum and tungsten represented by the following general formula III:

$[D]q\ Hr\ [XMo_5\ W_{6}-S\ O_{24}]$

wherein D stands for an alkali metal atom, an ammonium group, an alkylammonium group, providing that the number of carbon atoms of the alkyl group is an integer in the range of 1 to 5, or a mixture thereof, X for iodine, chromium, cobalt platinum, tellurium, nickel, manganese, aluminum, gallium, zinc, iron, or copper, q for an integer in the range of 0 to 16, r for an integer in the range of 0 to 16, and s for an integer in the range of 0 to 6.

This invention also discloses an antineoplastic drug wherein the heteropoly acid salt of molybdenum or tungsten is in the form of an aqueous solution. Further this invention discloses an antineoplastic drug wherein the aqueous solution is stable at a pH value in the range of 4 to 8. This invention further discloses an antineoplastic drug wherein the heteropoly acid salt is represented by the formula, $Na_5\ [IMo_6\ O_{24}] \bullet 34H_2O$. This invention further discloses an antineoplastic drug wherein the heteropoly acid salt is represented by the formula, $[NH_4]_3\ H_6\ [CoMo_6\ O_{24}] \bullet 7H_2O$. This invention further discloses an antineoplastic drug wherein the heteropolyacid salt is represented by the formula, $NA_3\ H_6\ [CrMo_6\ O_{24}] \bullet 8H_2O$. This invention discloses an antineoplastic drug wherein the tumors subjected to the treatment therewith are solid tumors.

Brief Description of Drawings

Fig. 1 and Fig. 2 are graphs each showing the effect manifested by the antineoplastic drug of this invention along the course of time,

Fig. 3 is an explanatory diagram concerning a mouse as a subject of an experiment,

Fig. 4 is an explanatory diagram illustrating the essential part of a dissected abdomen of a mouse, and

Fig. 5 is a schematic diagram illustrating transplantation of tumors to a region under the renal coating.

Best Mode for carrying out the Invention

The polymolybdate to be used in the antineoplastic drug of this invention is a compound represented by the general formula I:

$$[A]m\ [Mo_x\ O_y\ H_z] \cdot \ell\ H_2O \qquad (I)$$

In this formula, A stands for

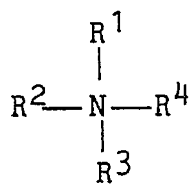

wherein $R^1$, $R^2$, $R^3$, and $R^4$ independently stand for hydrogen atom or a lower alkyl group such as, for example, an alkyl group of 1 to 6, preferably 1 to 3, carbon atoms, which may be identical or not, preferably $R^1$ to $R^4$ are not invariably hydrogen atoms as in a polyalkylammonium molybdate, or an alkyl metal atom or the mixture of an alkali metal with other alkali metal, hydrogen atom, an ammonium group, or an alkylammonium group, providing that the number of atoms of the alkyl group is in the range of 1 to 3, m for an integer in the range of 1 to 16, n for an integer such that the negative charge, xn, assumes the value of -m, x for an integer in the range of 2 to 36, y for an integer in the range of 4 to 112, z for an integer in the range of 0 to 24, and $\ell$ for an integer in the range of 0 to 36. Preferably in this formula, $R^1$ is hydrogen atom, $R^2$, $R^3$, and $R^4$ are independently hydrogen atom or an alkyl group of 1 to 3 carbon atoms, except for the case in which $R^2$ to $R^4$ are invariably hydrogen atoms, m is an integer in the range of 2 to 8, n an integer in the range of 2 to 8, x an integer in the range of 2 to 20, y an integer in the range of 10 to 50, z an integer in the range of 0 to 10, and $\ell$ an integer in the range of 1 to 10.

The polymolybdate to be used in the antineoplastic drug with this invention is a compound represented by the general formula II mentioned above. Further, the heteropoly acid salt to be used in the antineoplastic drug according with this invention is a compound represented by the general formula III.

$[D]_qH_r[XMo_s W_{6-s} O_{24}]$

In this formula, D stands for an alkali metal atom, an ammonium group, an alkylammonium group, providing that the number of carbon atoms of the alkyl group is an integer in the range of 1 to 5, or a mixture thereof, X for iodine, chromium, cobalt, platinum, tellurium, nickel, manganese, aluminum, gallium, zinc, iron, or copper, q for an integer in the range of 0 to 16, r for an integer in the range of 0 to 16, and s for an integer in the range of 0 to 6. Then, X desirably stands for an element selected from among heptavalent iodine, bivalent and trivalent cobalts, hexavalent and heptavalent telluriums, bivalent and tetravalent nickels, bivalent and tetravalent manganeses, bivalent zinc, bivalent copper, and bivalent and trivalent irons. Preferably in the formula, q is an integer in the range of 0 to 6 and r for an integer in the range of 0 to 6.

The heteropolyacid salt to be used in the present invention may be heteropolyacid salts of molybdenum and/or tungsten. Preferably, the heteropolyacid salt is desired to have molybdenum atom and/or tungsten atom coordinated thereto in the Anderson type.

The term "Anderson type" as used herein refers to a configuration having six molybdenum and/or tungsten atoms coordinated with the center of iodine, chromium, cobalt, platinum, tellurium, nickel, manganese, aluminum, zinc, or copper atom within the molecule.

Hidetake Kondo has published his report concerning heteropolymolybdates, the method of synthesis thereof, various analytical data, the configuration, and the Anderson type [Hidetake Kondo, Acta Crystallogr., Section B 36, 661-664 (1980)].

Alvin Perloff has published his report covering $Na_3$ H6 $[CrMo_6 O_{24}] 8H_2O$ , a heteropolymolybdate, the method of synthesis thereof, various analytical data, the configuration, and the Anderson type [Alvin Perloff, Inorg. Chem., 9 (10), 2228 (1970)].

The polymolybdate to be used in the present invention is one sort of isopoly acid which is a cluster compound. Specifically, it is an oxo acid ion polynucleous complex of a configuration in which basic units each generally having four or six oxygen atoms coordinated to a molybdenum and/or tungsten atom are bound to one another through the medium of edges or apexes. The general characteristics of such isopoly acid ions are as follows, for example.

(1) The isopoly acid ions have ion sizes in the range of 6 to 25 Angstroms and molecular weights in the range of 1,000 to 10,000.

(2) They are mostly obtained in the form of crystals and exhibit high degrees of solubility in water and polar solvents.

(3) They have large numbers of hydrated molecules.

(4) They are strong oxidizing agents (acting as multi-electron pooling agents).

The poly acid ions in a solid state and in the form of a solution possess the nature of effecting a photooxidation-reduction reaction and are earnestly expected to find utility in display function devices. The magnitude of the reactivity of these compounds implies the compounds possession of an ability of physiological activity. As the result of numerous studies, it has been found that the heteropoly acid salt of molybdenum and/or tungsten possesses a highly conspicuous antineoplastic effect. It shows a dramatic effect on such solid tumors as breast cancer; lung cancer, and sarcoma which will pose the most serious problem in the future. It manifests decisively low toxicity as compared with aminonitrosourea (ACNU) which is now in popular use.

The polymolybdate to be used in the present invention may be any of polymolybdates. Preferably, it is desired to be a polymolybdate of the side-on type containing a molybdenum center having oxygen molecules coordinated thereto.

The term "side-on type" as used herein refers to a compound containing a peroxide of the type,

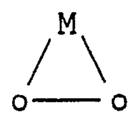

,

wherein M stands for a Mo atom in the molecular unit thereof.

I. Persdoller et al. have published a report covering $K_6[Mo_7O_{22}(O_2)_2] \bullet 10H_2O$, a polymolybdate, the method for synthesis thereof, various analytic data, the structure, and the side-on type [I. Persdoller, L. Trysberg, and R. Stomberg, Acta Chemica Scandinavica, A40, 335-343 (1986)]. They have further published a report on $(NH_4)_8[Mo_{10}O_{22}(O_2)_{12}] \bullet 16H_2O$ [I. Persdoller, L. Trysberg, and R. Stomberg, ibid, A40, 83-90 (1986)] and a report on $K_6[Mo_5O_{10}(O_2)8] \bullet 5H_2O$ [I . Persdoller, L trysberg, and R. Stomberg, ibid, A40, 1-7

(1986)].

The polymolybdates of this kind include those compounds which are represented by the following formulas, for example.

$[NH_3C_3H_7(iso)]_6\ [Mo_7O_{24}] \bullet 3H_2O$ (Compound A)
$[NH_3C_3H_7(iso)]_4\ [Mo_{13}O_{40}]\ 1/3 \bullet$
$[Mo_{12}O_{40}H_2]\ 2/3$ (Compound B)
$[NH_3C_3H_7(iso)]_6\ [Mo_7O_{24}H]$ mixture (Compound C)
$[NH_4]_6\ [Mo_7O_{24}]\ .\ 2H_2O$ (Compound D)
$[NH_3C_3H_7(iso)]_6\ [H_2Mo_8O_{28} \bullet 3H_2O$
$[NH_3C_3H_7]_6\ [Mo_7O_{24}] \bullet 3H_2O$
$[NH_3CH_3]_8\ [Mo_{10}O_{34}] \bullet 2H_2O$
$[NH(C_2H_5)_3]_3\ (H_3O)\ [Mo_8O_{26}] \bullet 2H_2O$
$[NH_2(CH_3)_2]_4\ [Mo_6O_{20}] \bullet 2H_2O$
$[NH_2(C_2H_5)_2]_2\ [Mo_3O_{10}] \bullet H_2O$
$K_6\ [Mo_7O_{24}] \bullet 4H_2O$ (Compound E),
$Na_6\ [Mo_7O_{24}] \bullet 4H_2O$ (Compound F),
$Li_6\ [Mo_7O_{24}] \bullet 4H_2O$ (Compound G),
$Rb_6\ [Mo_7O_{24}] \bullet 4H_2O$,
$Cs_6\ [Mo_7O_{24}] \bullet 4H_2O$ (Compound H)
$K_3\ Na_3\ [Mo_7O_{24}] \bullet 4H_2O$
$K_4 \bullet H2\ [Mo_7O_{24}] \bullet 4H_2O$
$K_4 \bullet (NH_4)_2\ [Mo_7O_{24}] \bullet {}_4H_2O$
$K_4\ [NH_3(iso)]_2\ [Mo_7O_{24}] \bullet 4H_2O\ K_4\ [Mo_{13}O_{40}]\ 1/3\ [Mo_{12}O_{40}H_2]\ 2/3$
$Na_4\ [Mo_{13}O_{40}]\ 1/3\ [Mo_{12}O_{40}H_2]\ 2/3$
$Li_4\ [Mo_{13}O_{40}]\ 1/3\ [Mo_{12}O_{40}H_2]\ 2/3$
$Rb_4\ [Mo_{13}O_{40}]\ 1/3\ [Mo_{12}O_{40}H_2]\ 2/3$
$Cs_4\ [Mo_{13}O_4O]\ 1/3\ [Mo_{12}O_{40}H_2]\ 2/3$
$K_6\ [Mo_7O_{24}H]$ mixture
$Na_6\ [Mo_7O_{24}H]$ mixture
$Li_6\ [Mo_7O_{24}H]$ mixture
$Rb_6\ [Mo_7O_{24}H]$ mixture
$Cs_6\ [Mo_7O_{24}H]$ mixture
$K_2 \bullet Na_3\ [Mo_7O_{24}H]$ mixture
$K_4 \bullet H_2\ [Mo_7O_{24}H]$ mixture
$K_4 \bullet (NH_4)_2\ [Mo_7O_{24}H]$ mixture
$K_4\ [NH_3(iso)]_2\ [Mo_7O_{24}H]$ mixture
$K_6\ [Mo_8O_{28}H_2]\ 2H_2O$
$Na6\ [Mo_8O_{28}H_2] \bullet 2H_2O$
$Li_6\ [Mo_8O_{28}H_2]\ .\ 2H_2O$
$Rb_6\ [Mo_8O_{28}H_2] \bullet 2H_2O$
$Cs_6\ [Mo_8O_{28}H_2] \bullet 2H_2O$
$K_3 \bullet Na_3\ [Mo_8O_{28}H_2] \bullet 2H_2O$
$K_4 \bullet H_2\ [Mo_8O_{28}H_2] \bullet 2H_2O$
$K_4 \bullet (NH_4)_2\ [Mo_8O_{28}H_2] \bullet 2H_2O$
$K_4\ [NH_3(iso)]_2\ [Mo_8O_{28}H_2] \bullet 2H_2O$
$K_8\ [Mo_{10}O_{34}] \bullet 2H_2O$,
$Na_8\ [Mo_{10}O_{34}]\ 2H_2O$
$Li_8\ [Mo_{10}O_{34}] \bullet 2H_2O$,
$Rb_8\ [Mo_{10}O_{34}] \bullet 2H_2O$
$Cs_8\ [Mo_{10}O_{34}] \bullet 2H_2O$
$K_3\ (H_3O)\ [Mo_8O_{26}] \bullet 2H_2O$
$Na_3\ (H_3O)\ [Mo_8O_{26}] \bullet 2H_2O$
$Li_3\ (H_3O)\ [Mo_8O_{26}] \bullet 2H_2O$
$Rb_3\ (H_3O)\ [Mo_8O_{26}] \bullet 2H_2O$
$Cs_3\ (H_3O)\ [Mo_8O_{26}] \bullet 2H_2O$
$K_4\ [Mo_6O_{20}] \bullet 2H_2O$,
$Na_4\ [Mo_6O_{20}] \bullet 2H_2O$
$Li_4\ [Mo_6O_{20}] \bullet 2H_2O$, $Rb_4\ [Mo_6O_{20}] \bullet 2H_2O$

$Cs_4$ [$Mo_6O_{20}$] • $2H_2O$
$K_2$ [$Mo_3O_{10}$] • $H_2O$,
$Na_2$ [$Mo_3O_{10}$] • $H_2O$
$Li_2$ [$Mo_3O_{10}$] • $H_2O$,
$Rb_2$ [$Mo_3O_{10}$] • $H_2O$ $Cs_2$ [$Mo_3O_{10}$] • $H_2O$ wherein iso stands for an isopropyl group.

The compounds of General Formula II include those listed below, for example.

$K_6$ [$Mo_7O_{22}(O_2)_2$] • $8H_2O$ (Compund I),
$Li_6$ [$Mo_7O_{22}(O_2)_2$] • $8H_2O$,
$Na_6$ [$Mo_7O_{22}(O_2)_2$] • $8H_2O$,
$Rb_6$ [$Mo_7O_{22}(O_2)_2$] • $8H_2O$,
$Cs_6$ [$Mo_7O_{22}(O_2)_2$] • $8H_2O$,
[$NH_3$ (iso)]$_6$ [$Mo_7O_{22}(O_2)_2$] • $5H_2O$ (Compund J),
$K_6$ [$Mo_7O_{23}(O_2)$] • $8H_2O$ (Compund K),
$Li_6$ [$Mo_7O_{23}(O_2)$] • $8H_2O$,
$Na_6$ [$Mo_7O_{23}(O_2)$] • $8H_2O$,
$Rb_6$ [$Mo_7O_{23}(O_2)$] • $8H_2O$,
$Cs_6$ [$Mo_7O_{23}(O_2)$] • $8H_2O$ and
[$NH_3$ (iso)]$_6$ [$Mo_7O_{23}(O_2)$] • $5H_2O$ (Compund L)
$K_6$ [$Mo_5O_{16}(O_2)_8$] • $5H_2O$
$(NH_4)_4$ [$Mo_3O_7(O2)_4$] • $2H_2O$
$K_5$ [$Mo_7O_{22}(O_2)_2(OH)$] • $6K_2O$
[$NH_4$]$_4$ [$Mo_8O_{24}(O_2)_2(OH)_2$] • $_4H_2O$ $K_4Mo_4O_{12}(O_2)_2$ [$NH_4$]$_8$ [$Mo_{10}O_{22}(O_2)_{12}$] • $10H_2O$ wherein iso stands for an isopropyl group.

The compounds of General Formula III include those listed below, for example.

$Na_5$ [$IMo_6O_{24}$] • $3_4H_2O$ (Compund M),
[$NH_4$]$_3$ $H_6$ [$CoMo6O_{24}$] • $7H_2O$ (Compund N),
$Na_3H_6$ [$CrMo_6O_{24}$] • $8H_2O$ (Compund O),
[$NH_3C_3H_7$(iso)]$_3$ $Na_3$ [$TeMo_6O_{24}$],
$Na_2(NH_4)_2H_6$ [$NiW_6O_{24}$],
[$NH_3C_3H_7$(iso)]$_8$ [$NiW_6O_{24}$],
$Na_3(NH_4)_3$ [$MnW_6O_{24}$],
$(NH_4)_4$ $H_6$ [$FeMo_6O_{24}$],
$Na_4H_6$ [$CuMo_6O_{24}$],
[$NH_3C_3H_7$(iso)]$_4H_6$ [$ZnMo_6O_{24}$],
$K_3H_6$ [$A\ell Mo_6O_{24}$],
[$NH_4$]$_3H_6$ [$GaMo_6O_{24}$]. wherein any formula containing "iso" refers to an isomer of the compound.

The antineoplastic drug according with this invention contains at least one of the compounds of General Formulas I to III mentioned above generally in a solid or liquid carrier, particularly in a liquid carrier. The compound according with this invention can be used in combination with a known active substance.

The antineoplastic drug of this invention can be administered for the antineoplastic purpose in any suitable manner possibly in the same manner as heretofore practised. When the compound mentioned above is non-orally, subcutaneously, intravenously, or intraperitonealy administered, the carrier for the antineoplastic drug is a pharmaceutically allowable aspectic liquid such as water or vegetable oil, especially water. Generally, the water as a carrier for an injection grade aqueous solution contains 0.1 to 50% by weight, preferably 1 to 20% by weight, of an active substance. The compound according with this invention can be also administered orally. It can be used advantageously for the therapy of lung cancer, for example, by being administered in the form of vapor or spray to the nose or the throat. The forms in which the antineoplastic drug is most advantageously administered orally include tablet, capsule, powder, solution, and suspension, for example. In this form of antineoplastic drug prepared for administration, the dosage of the compound of this invention for adult is in the range of 50 to 400 mg, preferably 100 to 200 mg, per day.

Now, the present invention will be described more specifically below with reference to working examples.

Examples 1 to 6 and Comparative Examples 1 to 5 (Evaluation by size of tumor)

By dissolving alkylammonium salt of polymolybdic acid [$NH_3C_3H_7$(iso)]$_6$ [$Mo_7O_{24}$] • $3H_2O$ (Compound A) and other compounds (Compounds B to D) severally in distilled water, aqueous solutions containing the compounds in a fixed concentration of 10 mg/ml were obtained. These aqueous solutions were administered to female BALB/C mice (five weeks old) to which methyl cholanthrene A tumor had been transplanted to test the compounds for antineoplastic activity. To be specific, methyl cholanthrene A was

transplanted to the mice in a ratio of 1.0 x $10^5$ cells per mouse and the aqueous solutions were continuously administered to the mice by intraperitoneal injection (at a dose of 100 mg/kg of body weight) for nine days. On elapse of 14 days following the start of the administration, the tumors in the mice were measured. The results were as shown in Table 1. For comparison, mice to which no antineoplastic .drug was administered and mice to which aminonitroso urea was administered were tested by following the procedure described above. The results were as shown in Table 1.

Table 1

| No. | Instance | Active compound | Dosage (mg/kg, ip × 9) | Change in body weight* (g) | Range of tumor size (mg) | Average weight of tumor (mg) | T/control | Number of tumorless mice /total number of mice |
|---|---|---|---|---|---|---|---|---|
| | Comparative Example 1 | Control (no drug) | - | +3.24 | 3430, 2448, 3971, 1476, 3166, 2723, 1884, 1688, 2126, 3350 | 2676.0±284.1 | - | 0/10 |
| 1 | Comparative Example 2 | aminonitrosourea | 5 | +1,58 (D1~D9) | 710, 1470, 1224, 817, 992, 2048, 716, 1303 | 1151.1±162.7 | 0.430 | 0/8 |
| | Example 1 | Compound A | 100 | +2.50 (D1~D9) | 635, 349, 499, 801, 304, 496, 525, 0 | 451.0±84.7 | 0.169 | 1/8 |
| | Comparative Example 3 | Control (no drug) | - | +2.41 | 2772, 4185, 2713, 2983, 2943, 2939, 2960, 2880, 2072, 2104 | 2885.1±183.1(10) | - | 0/10 |
| 2 | Example 2 | Compound A | 200,ip×9 D1-D9 | +1.81 | 218, 1152, 2031, 462, 1280, 1569, 1932, 1050 | 1211.8±227.6(8)**** | 0.424 | 0/8 |
| | | Compound A | 100,ip×9 D1-D9 | +1.97 | 777, 307, 694, 1823*, 482, 827, 161, 659, 886, 282 | 563.9±99.1(9)**** | 0.198 | 0/10 |
| | | Compound A | 50,ip×9 D1-D9 | +1.81 | 764, 1001, 423, 1838, 2150, 2364, 129, 483, 947, 1708 | 1182.1±246.8(10)**** | 0.414 | 0/10 |
| | Comparative Example 4 | Control (no drug) | - | +)2.76 | 2183, 2828, 2758, 2565, 2275, 3359, 2909, 2738, 2172, 2723 | 2575.5±94.6(10) | - | 0/17 |
| | Comparative Example 5 | aminonitrosourea | 5 | +1,58 (D1~D9) | 710, 1470, 1224, 817, 922, 2048, 716, 1303 | 1151.1±162.7 | 0.430 | 0/8 |
| 3 | Example 3 | Compound A | 50,ip×9 D1-D9 | +1.68 | 1141, 1438, 1969, 2078, 1936, 928, 1280, 2079 | 1606.0±163.5(8)**** | 0.624 | 0/8 |
| | Example 4 | Compound B | 25,ip×9 D1-D9 | -0.36 | 2117, 1991, 1688, 1458, 2256, 1655, 1016, 2093, 1505 | 1753.1±132.4(9)**** | 0.681 | 0/9 |
| | Example 5 | Compound C | 25,ip×9 D1-D9 | -2.19 | 1332, 1000, 1840, 1101, 1809, 1904, 975, 1911, 866, 1434 | 1425.2±130.6(10)**** | 0.553 | 0/10 |
| | Example 6 | Compound D | 100,ip×9 D1-D9 | +1.20 | 1818, 1962, 2432, 1382, 2449, 908, 1438, 1960 | 1790.5±189.3(8)**** | 0.695 | 0/8 |

* Body weight of mouse 13 days after transplantation of tumor

It is clearly noted from the results of the test that in all the cases of intraperitoneal administration, the active compounds of this invention manifested an effect of suppressing tumorous hyperplasia. In one of these cases, perfect extinction of tumor was observed. Thus, the compounds of this invention proved to be distinctly superior to aminonitrosourea used for comparison. As concerns toxicity, while the compound for comparison showed the largest allowable dose at 5 mg/kg.ip, the active compound (Compound A, for example) of this invention continuously administered at a dose of 200 mg/kg.ip for nine days to mice brought about a desirable increase of body weight in the mice, a fact which indicates that the compounds possessed very low toxicity. Other compounds according with this invention but not shown in the table manifested a similar effect in suppressing tumorous hyperplasia.

Examples 7 to 11 and Comparative Examples 6 to 9

(Evaluation by extension of period of survival)

The aforementioned compounds, A to D, were severally dissolved in distilled water to obtain aqueous solutions containing the compounds in a fixed concentration of 10 mg/ml. To the female BALB/C mice (five weeks old) to which methyl cholanthrene A had been transplanted in a ratio of $1.0 \times 10^5$ cells per mouse, the aqueous solutions were continuously administered at a dose of 100 mg/kg of body weight for nine days by intraperitoneal injection to determine the mices' periods of survival. The results were as shown in Table 2. For comparison, mice to which no antineoplastic drug was administered and mice to which aminonitroso urea was administered were similarly tested. The results were as shown in Table 2.

Table 2

| No. | Instance | Active compound | Dose (mg/kg, ip × 9) | Therapeutic planning (D1~D9) | Change in body weight* (g) | Range of number of days of survival (days) | Average number of days of survival (days) | T/control | Number of toxically killed mice/total number of mice |
|---|---|---|---|---|---|---|---|---|---|
| | Comparative Example 6 | Control (no drug) | - | - | +3.24 | 20, 24, 24, 27, 27, 27, 27,27, 27, 29 | 5.9±0.8 | - | 0/10 |
| 1 | Comparative Example 7 | aminonitrosourea | 5 | D1~D9 | +1.58 | 29, 34, 34, 34, 35, 38, 41, 41 | 35.8±1.4 | 138.0 | 0/8 |
| | Example 7 | Compound A | 100 | D1~D9 | +2.50 | 31, 34, 34, 43, 43, 49, 49, 55 | 42.3±3.0 | 163.1 | 0/8 |
| | Comparative Example 8 | Control (no drug) | - | - | +2.76 | 16, 18, 21, 21, 23, 23, 24, 25, 26, 28 | 22.5±1.1(10) | - | 0/10 |
| | Comparative Example 9 | aminonitrosourea | 5 | D1~D9 | +1.58 | 23, 24, 27, 28, 29, 29, 31, 33 | 28.0±3.1(9) | 124.5 | 0/8 |
| 2 | Example 8 | Compound A | 50 | D1~D9 | +1.68 | 24, 24, 28, 33, 33, 35, 35, 42 | 31.8±2.2(8) | 131.8 | 0/8 |
| | Example 9 | Compound B | 25 | D1~D9 | -0.36 | 24, 26, 26, 26, 26, 28, 33, 33, 42 | 29.3±1.9(9) | 130.4 | 0/9 |
| | Example 10 | Compound C | 25 | D1~D9 | -2.19 | 21, 23, 26, 28, 28, 33, 33, 33, 35, 42 | 30.2±2.0(10) | 134.2 | 0/10 |
| | Example 11 | Compound D | 100 | D1~D9 | +1.20 | 21, 25, 33, 33, 33, 35, 43, 43 | 33.3±2.7(8) | 133.3 | 0/8 |

It is clearly noted from the results of test given above that the active compound according with this invention (Compound A, for example), in terms of period of survival, manifested a significant effect as evinced by the result, T/control = 63.1%, and proved to be superior to aminonitroso amine. Other compounds according with this invention but not shown in the table manifested similar effects in elongation of life.

Example 12

A patch of human breast cancer [cancer cells recommended by National Cancer Institute (NCI) of U.S.A.] (MX-1) measuring approximately 2 x 2 mm in area was transplanted to female nude mice 5 weeks old. For 10 days following the 17th day of the transplantation, an aqueous solution containing 1% by weight of the aforementioned isopropylammonium polymolybdate (Compound A) was administered continuously at a dose of 100 mg/kg or 200 mg/kg once per day by intraperitoneal injection (ip). After the stop of the administration, the volume of tumor was measured daily for 15 or 20 days to evaluate the effect of the compound. With either of the dose of 100 mg/kg and the dose of 200 mg/kg, significant suppression of hyperplasia began to appear around the 6th day of administration of ip (x 10). Even after the stop of the administration (25th or 30th day), a distinct suppression of cancerous hyperplasia of about 70% was retained. Generally, by the NCI standard, a ratio of suppression exceeding 50% is rated as effective. Thus, the compound was confirmed to possess a very strong effect. The results were as shown in Table 3, Table 4, Fig. 1, and Fig. 2. For comparison, similar mice to which the human cancer was transplanted and the aqueous solution was not administered were tested by following the procedure described above. The results were as shown in Table 3, Table 4, Fig. 1, and Fig. 2.

Table 3

| Number of days after transplantation of tumor | Control | | Compound A (100 mg/kg) | | |
|---|---|---|---|---|---|
| | Body weight (g) | Volume of tumor ($mm^3$) | Body weight (g) | Volume of tumor ($mm^3$) | T/C (%) |
| 0 day | 22.2±1.50 | 326.5±46.3 | 19.7±0.73 | 278.3±22.0 | - |
| 1 day | 22.6±1.42 | 421.3±50.4 | 20.4±0.86 | 283.3±50.8 | 67.3 |
| 2 day | 22.8±1.62 | 516.3±71.7 | 20.5±0.74 | 302.3±58.6 | 58.6 |
| 3 day | 23.0±1.74 | 536.8±77.2 | 20.6±0.77 | 324.7±123.5 | 60.5 |
| 4 day | 24.4±0.34 | 603.3±73.5 | 20.6±0.65 | 366.3±121.7 | 60.7 |
| 5 day | 24.7±0.39 | 716.3±117.4 | 20.5±0.69 | 413.7±168.8 | 57.8 |
| 6 day | 23.4±1.40 | 829.0±70.3 | 20.4±0.74 | 366.0±121.3 | 44.2** |
| 7 day | 24.1±1.05 | 908.0±71.9 | 20.5±0.57 | 302.7±173.5 | 33.3** |
| 9 day | 24.1±0.88 | 1076.0±91.7 | 19.8±0.47 | 397.3±226.9 | 36.9* |
| 10 day | 24.4±0.87 | 1370.0±115.6 | 20.7±0.57 | 406.7±202.6 | 29.7*** |
| 15 day | 24.5±0.66 | 2199.0±413.0 | 20.0±1.07 | 734.0±524.0 | 33.3* |
| 19 day | 25.2±0.47 | 2656.0±227.3 | 19.5±1.40 | 994.0±613.0 | 37.4* |
| 21 day | 25.6±0.58 | 3430±71.0 | 19.6±2.40 | 1039.0±646.0 | 30.0* |
| 25 day | 25.7±0.96 | 3984.0±450.2 | 19.2±2.57 | 1301.0±912.3 | 32.6* |

T.V. = $1/2 \times L \times W^2$ ($mm^3$)   N=4 (Control), 3 (Compound A)
* $p<0.05$,  ** $p<0.02$,  *** $p<0.01$

Table 4

| Number of days after transplantation of tumor | Control | | Compound A (200 mg/kg) | | |
|---|---|---|---|---|---|
| | Body weight (g) | Volume of tumor ($mm^3$) | Body weight (g) | Volume of tumor ($mm^3$) | T/C (%) |
| 1 day | 20.2±0.77 | 491.8±68.9 | 19.3±0.89 | 371.8±10.8 | |
| 2 day | 20.9±0.60 | 703.5±95.0 | 20.1±0.82 | 439.8±56.9 | |
| 4 day | 21.0±0.69 | 835.0±109.5 | 19.5±1.05 | 518.8±54.7 | 62.1* |
| 5 day | 21.4±0.60 | 1092.0±165.5 | 19.4±1.00 | 610.5±15.2 | 55.9* |
| 6 day | 20.8±0.60 | 1043.0±7.00 | 19.1±1.08 | 656.8±25.7 | 62.9**** |
| 7 day | 21.1±0.86 | 1394.0±157.9 | 19.4±1.05 | 759.3±84.3 | 54.5** |
| 8 day | 21.1±0.86 | 1466.8±160.4 | 19.4±1.05 | 852.0±126.7 | 58.1* |
| 9 day | 21.2±0.81 | 1641.5±193.9 | 19.6±1.24 | 695.7±29.6 | 42.4*** |
| 10 day | 21.1±0.79 | 1814.3±247.8 | 19.3±1.21 | 759.7±21.1 | 41.9** |
| 11 day | 21.7±0.59 | 2078.3±284.4 | 18.7±1.32 | 816.7±16.7 | 39.3** |
| 13 day | 21.2±1.00 | 2681.8±235.1 | 18.9±1.31 | 1070.0±41.0 | 39.9*** |
| 16 day | 21.4±0.97 | 3218.0±411.1 | 18.7±1.58 | 1110.0±21.0 | 34.5*** |
| 18 day | 21.7±0.93 | 4255.0±538.0 | 18.3±2.00 | 1446.0±75.0 | 33.9*** |
| 19 day | 22.1±0.94 | 4717.3±548.8 | 18.4±2.19 | 1405.0±159.8 | 29.9*** |
| 22 day | 22.5±0.84 | 5117.5±652.2 | 18.2±2.41 | 1583.3±145.2 | 30.9*** |
| 25 day | 22.5±0.88 | 5845.3±799.3 | 17.2±2.96 | 1708.0±243.2 | 29.2*** |
| 30 day | 22.9±1.14 | 7466.4±930.3 | 17.7±3.13 | 2033.7±430.9 | 27.2*** |

* $p<0.05$, ** $p<0.02$, *** $p<0.01$ **** $p<0.001$

Test for acute toxicity

Compound A showed no toxicity when it was administered to BALB/C mice at a dose of 3,600 mg/kg or to nude mice at a dose of 2,000 mg/kg ip.

Efficacy

The ammonium salts of polymolybdic acid according with the present invention significantly suppressed hyperplasia of methyl cholanthrene A tumor and manifested a significant effect of elongation of life even during the period of survival. It was clearly shown that they significantly suppressed the hyperplasia of human breast cancer and manifested a conspicuous effect on solid cancer. Examples 13 to 16 and Comparative Examples 10 and 11

(Evaluation by weight of tumor)

Compound E, an alkali metal salt of polymolybdic acid (Example 13), and other compounds (Compounds F to H) mentioned above were severally dissolved in distilled water to obtain aqueous solutions containing the compounds in a fixed concentration of 10 mg/ml. These aqueous solutions were administered to female BALB/C mice (five weeks old) to which methyl cholanthrene A tumor had been transplanted to test the compounds for antineoplastic activity. To be morespecific, the methyl cholanthrene A was transplanted to the mice in a ratio of 1.0 x $10^5$ cells per mouse and the aqueous solution was continuously administered (at a dose of 100 mg/kg of body weight) for nine days by intraperitoneal injection. On elapse of 14 days after the stop of the administration, the tumors in the mice were weighed. The results were as shown in Table 5. For comparison, mice to which the aqeuous solution was not administered and the mice to which aminonitrosourea was administered were similarly tested. The results were as shown in Table 5.

Table 5

| No. | Instance | Active compound | Dosage (mg/kg, ip x 9) | Change in body weight* (g) | Range of tumor size (mg) | Average weight of tumor (mg) | T/control | Number of tumorless mice/ total number of mice |
|---|---|---|---|---|---|---|---|---|
| | Comparative Example 10 | Control (no drug) | - | (+)2.76 | 2183, 2828, 2758, 2565, 2775, 3359, 2909, 2738, 2172, 2723, 2756, 2176, 2995, 2552, 2563, 2541, 1690 | 2575.5±94.6(17) | - | 0/17 |
| | Comparative Example 11 | aminonitrosourea | 5,ip×9 D1-D9 | +1,58 | 710, 1470, 1224, 817, 922, 2048, 716, 1303 | 1151.1±162.7 | 0.447 | 0/8 |
| | Example 13 | Compound E | 100,ip×9 D1-D9 | (+)0.20 | 1568, 350, 1997, 1259, 968, 912, 1587, 1611 | 1281.6±184.2(8)** | 0.498 | 0/8 |
| | Example 14 | Compound F | 100,ip×9 D1-D9 | (+)1.02 | 1389, 1541, 1569, 1781, 2031, 1853, 1233, 1345 | 1592.8±257.6(8)** | 0.618 | 0/8 |
| | Example 15 | Compound G | 100,ip×9 D1-D9 | (+)0.88 | 1518, 1731, 1865, 1392, 1481, 1960, 2008, 2125 | 1760±254.6(8)** | 0.683 | 0/8 |
| | Example 16 | Compound H | 100,ip×9 D1-D9 | (+)2.05 | 1820, 1931, 1785, 2031, 1644, 1381, 1056, 1148 | 1599.5±340.6(8)** | 0.621 | 0/8 |

* Body weight of mouse measured 13 days after tumor transplantation.
** $p<0.001$

It is clearly noted from the results of test given above that the active compounds of the present invention showed an effect in suppressing hyperplasia of tumor in all the cases of intraperitoneal administration. The effects the compounds showed were virtually equal to the effect brought about by aminonitrosourea. As regards toxicity, while the compound used for comparison showed the largest allowable dose of 5 mg/kg. ip, the compound according with this invention (Compound E, for example) continuously administered at a dose of 100 mg/kg. ip for nine days showed very slight toxicity as evinced by a desirable increase of body weight. Other compounds according to this invention but now shown in the table exhibited similarly high effects in suppressing the hyperplasia of tumor.

Examples 17 to 20 and Comparative Examples 12 and 13

(Evaluation by elongation of period of survival)

The aforementioned active compounds, E to H, were severally dissolved in distilled water to obtain aqueous solutions containing the compounds in a fixed concentration of 10 mg/ml. To female BALB/C mice (five weeks old) to which methyl cholanthrene A had been transplanted in a ratio of 1.0 x $10^5$ cells per mouse, the aqueous solutions were continuously administered at a dose of 100 mg/kg of body weight for nine days by intraperitoneal injection to determine the mices periods of survival. The results were as shown in Table 6. For comparison, mice to which no aqueous solution was administered and mice to which aminonitrosourea was administered were similarly tested. The results were as shown in Table 6.

Table 6

| No. | Instance | Active Compound | Dosage (mg/kg, iD × 9) | Therapeutic planning (D1~D9) | Change in body weight* (g) | Range of number of days of survival (days) | Average number of days of survival (days) | Ratio of prolonging life(%) | Number of toxically killed mice/total number of mice |
|---|---|---|---|---|---|---|---|---|---|
| | Comparative Example 12 | Control (no drug) | - | - | (+) 2.76 | 16, 18, 21, 21, 23, 23, 24, 25, 26, 28 | 22.5±1.1(10) | - | 0/10 |
| | Comparative Example 13 | Aminonitro sourea | 5 | D1~D9 | + 1.58 | 23, 24, 27, 28, 29, 29, 31, 33 | 28.0±3.1(9) | 24.4 | 0/8 |
| | Example 17 | Compound E | 100 | D1~D9 | (+) 0.20 | 26, 28, 33, 35, 36, 42, 42, 42 | 35.5±2.2(8)**** | 57.8 | 0/8 |
| | Example 18 | Compound F | 100 | D1~D9 | (+) 1.20 | 25, 28, 33, 35, 37, 39, 41, 42 | 35.0±5.7(8)**** | 55.6 | 0/8 |
| | Example 19 | Compound G | 100 | D1~D9 | (+) 0.88 | 24, 26, 27, 30, 34, 35, 27, 40 | 31.6±5.4(8****) | 40.4 | 0/8 |
| | Example 20 | Compound H | 100 | D1~D9 | (+) 2.05 | 25, 28, 29, 30, 33, 38, 39, 41 | 32.9±5.5(8)**** | 46.2 | 0/8 |

*The weight of mouse measured 13 days after tumor transplantation.

****$P < 0.001$

It is clearly noted from the results of test given above that the active compound according with this invention (Compound E, for example) exhibited a significant effect in elongation of life as evinced by a ratio of elongation of 57.8% and proved to be superior to aminonitrosourea in terms of the period of survival. Other compounds according with this invention but not shown in the table showed a similarly high effect in elongation of life.

Examples 21 to 24 and Comparative Examples 14 and 15

(Evaluation by size of tumor)

A polymolybdate, $K_6[Mo_7O_{22}(O_2)_2]8H_2O$ (Compound I), and the aforementioned other compounds (Compounds J to L) were severally dissolved in distilled water to obtain aqueous solutions containing the compounds in a fixed concentration of 10 mg/ml. To female C3H/He mice (five weeks old) to which MM-46 breast cancer had been transplanted, the aqueous solutions were administered to test the compounds for antineoplastic activity. To be more specific, the MM-46 breast cancer was transplanted to the mice at a ratio of $5.0 \times 10^5$ cells per mouse and the aqueous solutions were continuously administered (at a dose of 100 mg/kg of body weight) by intraperineal injection for nine days. On elapse of 14 days after the stop of the administration, the size of tumor in the mice was measured. The results were as shown in Table 7. For comparison, mice to which the aqueous solution was not administered and mice to which aminonitroso urea was administered were similarly tested. The results were as shown in Table 7.

Table 7

| Example | Active Compound | Dosage (mg/kg, ip × 9) | Change in body weight* (g) | Range of tumor size (mg) | Average weight of tumor (mg) | T/Control | Number of tumorless mice/total number of mice |
|---|---|---|---|---|---|---|---|
| Comparative Example 14 | Control (no drug) | - | (+)2.41 | 2772, 4185, 2713, 2983, 2943, 2939, 2960, 2880, 2072, 2104 | 2885.1±183.1(10) | - | 0/10 |
| Comparative Example 15 | Aminonitrosourea | 5, ip × 9 | (+)1.03 | 1523, 1548, 1439, 1244, 1651, 1131, 1043, 1335 | 1364.3±200.0 | 0.473 | 0/8 |
| Example 21 | Compound I | 100, ip × 9 | (+)2.45 | 1585, 1607, 283, 832, 634, 1169, 757, 696, 2099, 1687 | 1134.9±184.5(10)**** | 0.397 | 0/10 |
| Example 22 | Compound J | 100, ip × 9 | (+)1.44 | 1158, 1127, 829, 455, 290, 0, 0, 0, 0, 0 | 385.1±152.3(10)**** | 0.135 | 5/10 |
| Example 23 | Compound K | 100, ip × 9 | (+)1.85 | 1381, 1523, 1039, 1122, 983, 789, 1258, 1021 | 1139.5±221.1(8)**** | 0.399 | 0/8 |
| Example 24 | Compound L | 100, ip × 9 | (+)1.38 | 939, 867, 1245, 1185, 1022, 1029, 1233, 1165 | 1085.6±133.2**** | 00.380 | 0/8 |

*The weight of mouse measured 10 days after tumor transplantation
****P<0.001

It is clearly noted from the results of the test that in all the cases of intraperitoneal administration, the active compounds of this invention manifested an effect of suppressing tumorous hyperplasia. In one of these cases, perfect extinction of tumor was observed. Thus, the compounds of this invention proved to be distinctly superior to aminonitrosourea used for comparison. As concerns toxicity, while the compound for comparison showed the largest allowable dose at 5 mg/kg.ip, the active compound (Compound I, for example) of this invention continuously administered at a dose of 100 mg/kg.ip for nine days to mice

brought about a desirable increase of body weight in the mice, a fact which indicates that the compounds possessed very low toxicity. Other compounds according with this invention but not shown in the table manifested a similar effect in suppressing tumorous hyperplasia.

Examples 25 to 28 and Comparative Examples 16 and 17

(Evaluation by elongation of period of survival)

The active compounds, I to L, mentioned above were severally dissolved in distilled water to obtain aqueous solutions containing the compounds in a fixed concentration of 10 mg/ml. To female C3H/He mice (five weeks old) to which the MM-46 breast cancer had been transplanted in a ratio of 1.0 x $10^5$ cells per mouse, the aqueous solutions were continuously administered at a dose of 100 mg/kg of body weight by intraperitoneal injection for nine days to determine the mice's periods of survival. The results were as shown in Table 8. For comparison, mice to which no aqueous solution was administered and mice to which aminonitrosourea was administered were similarly tested. The results were as shown in Table 8.

Table 8

| Example | Active Compound | Dosage (mg/kg, ip × 9) | Therapeutic planning (D1~D9) | Change in body weight* (g) | Average weight of tumor after 14 days (mg) | Range of number of days of survival (days) | Average number of days of survival (days) | Ratio of elongation of life (5) |
|---|---|---|---|---|---|---|---|---|
| Comparative Example 16 | Control (no drug) | - | | (+)2.41 | 2885±183(10) | 10,10, 19, 19, 14, 19, 19, 19, 22 | 16.5±1.3(10) | - |
| Comparative Example 17 | Aminonitrosourea | 5, ip × 9 | D1~D9 | (+)1.03 | 1364.3±200.0 | 28, 30, 33, 29, 27, 26, 31, 29 | 29.1±2.1(8) | 76.4 |
| Example 25 | Compound I | 100, ip × 9 | D1~D9 | (+)2.45 | 1135 ±185(8)**** | 40, 24, 32, 32, 29, 29, 29, 50 | 33.1±2.9(8)**** | 100.8 |
| Example 26 | Compound J | 100, ip × 9 | D1~D9 | (+)1.44 | 385±152 (8)**** | 26, 26, 29, 44, 50, 50, 50, 50 | 40.6±4.1(6)**** | 146.2 |
| Example 27 | Compound K | 100, ip × 9 | D1~D9 | (+)1.85 | 1140± 221(8)**** | 28, 29, 31, 31, 33, 34, 35, 36 | 32.1±2.7(8)**** | 94.5 |
| Example 28 | Compound L | 100, ip × 9 | D1~D9 | (+)1.38 | 1086 ± 132(8)**** | 27, 29, 30, 30, 31, 34, 37, 39 | 32.1±3.9(8)**** | 94.5 |

*Body weight of mouse 13 days after transplantation of tumor.

****P<0.001

It is clearly noted from the results of test shown above that the active compound according with the present invention (Compound I, for example) manifested a significant effect in elongation of life as evinced by a ratio of elongation of life of 100.8% and proved to be superior to aminonitrosourea in terms of period of survival. Other compounds according with the present invention but not shown in the table showed similar effects elongation of life.

Examples 29 and 30 and Comparative Examples 18 to 20

A patch of human breast cancer [cancer cells recommended by National Cancer Institute (NCI) (MX-1)] measuring approximately 1 x 1 mm in area was transplanted to each of female ICR/cd-1 mice (18 to 20 g) five to six weeks old (SRC method). This SRC method will be described briefly with reference to the accompanying drawing. Fig. 3 is an explanatory diagram of a mouse I as the subject of test. In Fig. 3, an incised part 3 is shown as extended from the costal end 2 of a mouse 1 to the abdominal cavity. Fig. 4 is an explanatory diagram illustrating the essential part of the incised abdomen of the mouse as the subject. As shown in Fig. 4, the skin 5 on one side of the incision was nipped with pincers 4 and pulled out to expose the stomach 6, the spleen 7, the kidney 8, and the subcutaneous fat 9 from inside the skin. Fig. 5 is a schematic diagram illustrating the scene of the transplantation of tumor to beneath the hepatic capsule. As shown in Fig. 5, a patch of tumor 12 was set in place in the leading end part of a slender venous tube 11 provided with a piston 10 and transplanted inside the capsule 13 of the kidney 8. This operation was carried out in the aseptic condition under an optical microscope. This SRC method has an advantage that it possesses a satisfactory correlation with the nude mouse method and it produces results quickly.

Then, an aqueous solution of 1% by weight of the compound I was continuously administered at doses of 100 and 200 mg/kg to the mice once daily for five days by intraperitoneal injection (ip). For seven days following the stop of the administration, the body weights of the mice and the volumes of their tumors were measured to evaluate the efficacy of the compounds. Compound J was evaluated by repeating the procedure described above.

The results were as shown in Table 9. For comparison, mice to which no aqueous solution was administered and mice to which 5-FU (5-fluorouracil) and HCFU (Mifurol) were administered were similarly tested. The results were as shown in Table 9.

Table 9

| Example | Active Compound | Dosage (mg/kg, ip(po)) | | Immediately before transplantation: Body weight (g) | Immediately before transplantation: Tumor (To) (omu)** | Body weight 7 days after transplantation: (W7) (g) | Body weight 7 days after transplantation: W7/Wo | Tumor 7 days after transplantation (T7)** (omu) | T7/To | T/C(%) |
|---|---|---|---|---|---|---|---|---|---|---|
| Comparative Example 18 | Control (no drug) | - | - | 18.4 | 15.0 | 20.7 | 1.125 | 17.5 | 1.167 | |
| | | | | 19.2 | 14.0 | 22.1 | 1.151 | 19.0 | 1.357 | |
| | | | | 20.6 | 14.0 | 23.8 | 1.155 | 16.5 | 1.107 | |
| | | | | 20.1 | 9.0 | 23.2 | 1.154 | 17.0 | 1.899 | |
| | | | | | | | 1.145±0.007 | | 1.380±0.177 | |
| Comparative Example 19 | 5-FU | 30 | ip(×5) | 18.9 | 18.0 | 20.2 | 1.069 | 16.5 | 0.917 | |
| | | | | 22.0 | 14.0 | 24.5 | 1.114 | 11.0 | 0.786 | |
| | | | | 20.2 | 15.5 | 23.0 | 1.139 | 13.0 | 0.839 | |
| | | | | 20.4 | 13.0 | 22.2 | 1.088 | 7.5 | 0.576 | 56.48* |
| | | | | | | | 1.102±0.015 | | 0.779±0.072 | |
| Comparative Example 20 | HCFU(Mifurol) | 129 | po(×5) | 19.6 | 14.0 | 21.3 | 1.087 | 13.0 | 0.929 | |
| | | | | 18.8 | 13.0 | 22.7 | 1.207 | 11.5 | 0.885 | |
| | | | | 21.2 | 13.5 | 21.8 | 1.028 | 10.5 | 0.741 | |
| | | | | 21.6 | 14.0 | 23.7 | 1.097 | 11.5 | 0.821 | |
| | | | | | | | 1.104±0.037 | | 0.844±0.040 | 61.15* |
| | | 257 | po(×5) | 19.2 | 14.5 | 21.7 | 1.109 | 13.0 | 0.896 | |
| | | | | 19.9 | 13.5 | 23.2 | 1.166 | 14.0 | 1.037 | |
| | | | | 18.0 | 15.0 | 18.2 | 1.011 | 12.5 | 0.833 | |
| | | | | 18.7 | 13.0 | 19.4 | 1.037 | 12.0 | 0.923 | |
| | | | | | | | 1.080±0.035 | | 0.922±0.042 | 66.82* |
| Example 29 | Compound I | 100 | ip(×5) | 18.0 | 12.0 | 21.0 | 1.061 | 10.5 | 0.875 | |
| | | | | 19.4 | 8.5 | 20.5 | 1.057 | 8.0 | 0.941 | |
| | | | | 19.6 | 12.5 | 21.4 | 1.092 | 9.5 | 0.760 | |
| | | | | 19.4 | 14.5 | 20.6 | 1.062 | 8.5 | 0.586 | |
| | | | | | | | 1.068±0.008 | | 0.790±0.077 | 57.28 8* |
| Example 30 | Compound J | 100 | ip(×5) | 19.1 | 11.5 | 22.3 | 1.168 | 10.5 | 0.913 | |
| | | | | 20.4 | 12.0 | 24.0 | 1.176 | 9.5 | 0.792 | |
| | | | | 21.4 | 12.5 | 24.3 | 1.136 | 10.5 | 0.840 | |
| | | | | 19.6 | 13.0 | 24.1 | 1.230 | 12.5 | 0.962 | |
| | | | | | | | 1.177±0.019 | | 0.876±0.037 | 63.53* |
| | | 200 | ip(×5) | 18.8 | 13.0 | 21.6 | 1.149 | 14.0 | 1.077 | |
| | | | | 19.5 | 9.0 | 23.2 | 1.190 | 11.0 | 1.222 | |
| | | | | 17.9 | 13.5 | 21.3 | 1.190 | 11.5 | 0.852 | |
| | | | | 19.7 | 12.5 | 23.0 | 1.168 | 11.0 | 0.880 | |
| | | | | | | | | | 1.007±0.087 | 73.02* |

The symbol, omu represents results of calculation of (minimum + maximum)/2

It is clearly noted from the results of Table 9 that the compound I of this invention showed strong antineoplastic activity. This effect was equal to the effect manifested by means of 5-FU administered at a dose of 30 mg/kg. The compound J of this invention showed the same degree of antineoplastic activity as that of HCFU.

Examples 31 to 33 and Comparative Examples 21 to 24

(Evaluation by size of tumor)

A heteropoly acid salt, $Na_5[IMo_6O_{24}]\bullet 34H_2O$ (Compound M), and other compounds (Compounds N and O ) were severally dissolved in distilled water to obtain aqueous solutions containing the salts in a fixed concentration of 10 mg/ml. To female BALB/C mice (five weeks old) to which methyl cholanthrene A tumor had been transplanted, the aqueous solutions were administered to test the compounds for antineoplastic activity. To be specific, the methyl cholanthrene A was transplanted to the mice at a ratio of 1.0 x $10^5$ cells per mouse and the aqueous solutions were administered (at a dose of 25 mg/kg and a dose of 100 mg/kg of body weight) for 9 consecutive days by intraperitoneal injection. On elapse of 14 and 21 days after the

stop of the administration, the sizes of tumors in the mice were measured. The results were as shown in Table 10 and Table 11. For comparison, mice to which no aqueous solution was administered and mice to which aminonitrosourea was administered were similarly tested. The results were as shown in Table 10 and Table 11.

## Table 10

| Example | Active Compound | Dosage (mg/kg, ip × 9) | Change in body weight after elapse of 11 days (g) | Average weight of tumor after elapse of 14 days (mg) ± SE(T/C) | Range of number of days survival days) | Average number of days of survival (days) ± SE | ILS (%) |
|---|---|---|---|---|---|---|---|
| Comparative Example 21 | Control (no drug) | - | (+)2.62 | 859.9±85.8(10) | 19, 26, 28, 28, 24, 28, 31, 21, 33, 35 | 26.30±1.51(10) | - |
| Example 31 | Compound M | 100(D1~D9) | (+)1.62 | 191.1±38.8(7)**** | 25, 42, 35, 74, 44, 42, 36 | 42.57±5.77(7)**** | 61.3 |
| Comparative Example 22 | ACNU | 2.5(D1~D9) | (+)1.48 | 347.9±63.8(8)**** (0.405) | 30, 35, 29, 36, 32, 30, 26 | 31.13±1.14(8) | 18 |

SE: Standard error

ACNU: Aminonitrosourea

To female BALB/C mice, methyl chloanthrene A was subcutaneously transplanted in a ratio of 1.0 × $10^5$ cells per mouse on 0 day, and compound M and ACNU were severally administered in prescribed amounts by means of intraperitoneal injection during the period of preservation.

The weights of tumor were measured 14 days after transplantation f tumor.

Significant error from the value obtained of the control group of tumor (*P<0.05, **P<0.02, ***P<0.01****P<0.001)

## Table 11

| Example | Active Substance | Dosage (mg/kg, ip × 9) | Change of body weigh t after elapse of 10 days (g) | Range of tumor weight after elapse of 14 days (mg) | Average weight of tumor (g) ± SE | Average weight of tumor T/C | Range of average tumor weight after elapse of 21 days (g) ± SE | Range of average tumor weight after elapse of 21 days T/C |
|---|---|---|---|---|---|---|---|---|
| Comparative Example 23 | Control (no drug) | - | (+)1.83 | 757, 1715, 1080, 812, 760, 509, 439, 434, 560, 394 | 747.0±127.4 (10) | - | 2302.0±209. 2(8) | - |
| Example 32 | Compound N | 25(D1~D9) | (+)0.56 | 207, 0,7, 1149, 442, 89, 271, 169, 571, 0 | 448.5±166.0 (10) | 0.600 | 693.6±144.3 (9)**** | 0.301 |
| Example 33 | Compound O | 25(D1~D9) | (+)1.22 | 556, 230, 298, 632, 394, 366, 240, 1361, 1004, 775 | 585.6±166.8 (10) | 0.784 | 1915.9±442. 2(10) | 0.832 |
| Comparative Example 24 | ACNU | 7.5(D1~D9) | (-)0.26 | 268, 287, 800, 230, 90, 213, 146, 379, 107 | 274.3±71.1( 9)*** | 0.367 | 1022.1±169. 0(9)**** | 0.444 |

SE: Standard error

ACNU: Aminonitrosourea

Significant error from the value obtained of the control group of tumor (*$P<0.05$, **$P<0.02$, ***$P<0.01$****$P<0.001$)

To BALB/C mice, methyl chranthrene A was subcutaneously transplanted in a ratio of $1.0 \times 10^5$ cells per mouse on 0 day, and compound N,O and ACNU were intraperitoneally injected for the period of preservation , i.e. consecutive days, on elapse of 24 hours following the transplantation of tumor.

It is clearly noted from the results of test given above that the active compounds of this invention showed an effect in suppressing hyperplasia of tumor in all of cases of intraperitoneal administration of the compounds. Even in two of these cases, perfect extinction of tumor was observed. The effects of these compounds were equal to or somewhat better than the effect brought about by aminonitrosourea. As regards toxicity, while the compound used for comparison showed the largest allowable dose of 7.5 mg/kg.ip, the active compound according with this invention (Compound M, for example) showed very slight toxicity as evinced by a satisfactory increase of body weight and proved even after it was administered at a dose of 100 mg/kg.ip for 9 consecutive days. Other compounds according with this invention, but not shown in the table, showed similar effects in suppressing hyperplasia of tumor.

Examples 34 and 35 and Comprative Examples 25 and 26

(Evaluation by elongation of period of survival)

The active compounds, M to 0, mentioned above were severally dissolved in distilled water to obtain aqueous solutions containing the compounds in a fixed concentration of 10 mg/ml. To be specific, to female BALB/C mice (five weeks old) to which methyl cholanthrene A had been transplanted in a ratio of $1.0 \times 10^5$ cells per mouse, the aqueous solutions were administered at a dose of 100 mg/kg of body weight by intraperitoneal injection for 9 consecutive days to determine the mice's periods of survival. The results were as shown in Table 10 and Table 12. For comparison, mice to which no aqueous solution was administered and mice to which aminonitrosourea was administered were similarly tested. The results were as shown in Table 10 and Table 12.

Table 12

| Example | Active Compound | Dosage (mg/kg, ip × 9) | Change in body weight after elapse of 10 days | Range of number of days of survival (days) | Average number r of days of survival (days) ± SE | ILS (%) | Number of mice of no tumor/total number of mice |
|---|---|---|---|---|---|---|---|
| Comparative Example 25 | Control (no drug) | - | (+)1.83 | 16, 20, 23, 26,0 26, 28, 28, 26, 28, 28 | 29,90 ± 1.30(100) | - | 0/10 |
| Example 34 | Compound N | 25(D1~D9) | (+)0.56 | 28, 33, 33, 29, 37, 40, 42, 31, >60>60 | 39.30 ± 3.73(10)**** | 57.0 | 2/10 |
| Example 35 | Compound O | 25(D1~D9) | (+)1.22 | 34, 5, 26, 33, 35, 42, 52, 26, 23, 30 | 35.10 ± 3.16(10)**** | 41.0 | 0/10 |
| Comparative Example 26 | ACNU | 7.5(D1~D9) | (-)0.26 | 37, 33, 41, 40, 33, 36, 40, 37, 33 | 36.67 ± 1.07(9)**** | 47.3 | 0/9 |

SE: Standard error

ACNU: Aminonitrosourea

Significant error from the value obtained of mice of control group (*$P<0.05$, **$P<0.02$, ***$P<0.01$****$P<0.001$)

To female BALB/C mice, methyl cholanthrene A was subcutaneously transplanted in a ratio of $1.0 \times 10^5$ cell per mouse on 0 day and compounds N and O and ACNU were intraperitoneally injected n prescribed amounts for 9 consecutive days after elapse of 24 hours following the transplantation of tumor.

The tumor less mouse was observed 60 days after transplantation of tumor.

%ILS: Ratio of elongation of life.

It is clearly noted from the results of test given above that the active compound according with this invention (Compound M, for example) showed a significant effect in elongation of life as evinced by a ratio of survival of 61.3% and proved to be superior to aminonitrosourea in terms of period of survival. Even in two of these cases, perfect extinction of tumor was observed. Other active compounds according with this invention, but not shown in the table, showed a similar effect in elongation of life.

Example 36 and Comparative Example 27

A patch of breast cancer [cancer cells recommended by National Cancer Institute (NCI) of the U.S.A.] (MX-1) measuring approximately 2 x 2 mm in area was transplanted to each of female nude mice five weeks old. To the mice, an aqueous solution of the aforementioned heteropoly acid salt (Compound M) was administered at a dose of 100 mg/kg once a day by intraperitoneal injection for 10 consecutive days on elapse of 17 days following the transplantation of tumor (ip). For 15 days following the stop of the administration, the volumes of tumor in the mice were measured to evaluate the effect of compound. At ip (x 10), significant suppression of hyperplasia appeared around the 4th day and a strong supression of hyperplasia of cancer was maintained even after 25 days following the completion of transplantation. The resutls were as shown in Table 13. For comparison, mice to which no aqueous solution was administered were similarly tested. The results were as shown in Table 13.

Table 13

| Number of days after transplantation of tumor (days) | Comparative Example 27 Control (no drug): Average change in body weight (g) Average ±SE | Comparative Example 27 Control (no drug): Average volume of tumor ($mm^3$) Average ±SE | Example 36 Compound M (100 mg/kg): Average change in body weight (g) Average ±SE | Example 36 Compound M (100 mg/kg): Average volume of tumor ($mm^3$) Average ±SE | T/C (%) |
|---|---|---|---|---|---|
| 1 | 19.9±0.34 | 312.0±8.00 | 19.3±0.34 | 333.2±25.0 | - |
| 2 | 20.7±0.37 | 445.2±16.5 | 19.6±0.50 | 404.7±16.5 | 90.9 |
| 3 | 21.0±0.38 | 575.0±25.0 | 19.6±0.61 | 510.0±38.8 | 88.6 |
| 4 | 20.9±0.44 | 669.0±27.8 | 19.1±0.72 | 575.0±14.4 | 85.9* |
| 5 | 20.8±0.32 | 743.0±32.1 | 18.5±0.63 | 555.2±44.7 | 74.7** |
| 6 | 21.0±0.19 | 746.0±32.1 | 18.5±0.61 | 565.5±36.7 | 76.1** |
| 7 | 20.7±0.38 | 957.7±38.1 | 17.9±0.53 | 616.7±20.3 | 64.3 **** |
| 8 | 20.7±0.43 | 1041.7±51.6 | 18.4±0.37 | 786.0±40.0 | 75.4*** |
| 9 | 20.9±0.54 | 1365.2±79.0 | 18.6±0.37 | 869.0±33.2 | 63.6*** |
| 10 | 21.4±0.39 | 1476.7±112.3 | 19.4±0.31 | 957.9±38.1 | 64.8*** |
| 11 | 21.5±0.37 | 1572.2±84.3 | 19.6±0.32 | 1092.7±32.4 | 69.5*** |
| 15 | 22.1±0.29 | 2492.7±265.8 | 20.5±0.11 | 1540.5±106.6 | 61.7** |
| 18 | 23.0±0.32 | 2957.7±247.7 | 21.0±0.21 | 1821.0±127.4 | 61.5*** |
| 21 | 23.4±0.18 | 3379.5±279.2 | 22.5±0.41 | 2182.0±148.6 | 64.5*** |
| 25 | 23.2±0.30 | 3659.2±247.6 | 22.7±0.46 | 2671.7±154.5 | 73.0** |

*P<0.05, **P<0.02, ***P<0.01, ****P<0.001

T=Groups to which compound was administered

C=Control group

SE=Standard error

Example 37 and Comparative Example 28

A patch of human lung cancer [Cancer cells recommended by National Cancer Institute (NCL) of the U.S.A.] (OAT) measuring approximately 2 x 2 mm in area was subcutaneously transplanted to each of female nude mice. An aqueous solution containing the aforementioned heteropoly acid salt (Compound M) in a concentration of 10 mg/ml was administered by intraperitoneal injection (ip) to the mice at a dose of 200 mg/kg once per day for 10 consecutive days following the 12th day of transplantation. For 15 days following the stop of the administration, the volumes of tumor in the mice were measured to evaluate the effect of compound. At ip (x 10), significant suppression of hyperplasia began to appear about the 4th day. Even after completion of the administration 25th day of the transplantation, strong suppression of hyperplasia of cancer of about 50% was maintained. Generally by the NCI standard, a ratio of suppression exceeding 50% is rated as effective. Thus, the compound was confirmed to be effective. The results were as shown in Table 14. For comparison, mice to which no aqueous solution was administered were similarly tested. The results were as shown in Table 14.

Table 14

Comparative Example 28

| Number of days after transplantation of tumor | | Body weight (g) Range | Body weight (g) Average value±SE | Volume of tumor ($mm^3$) Range | Volume of tumor ($mm^3$) Average value±SE |
|---|---|---|---|---|---|
| Tumor transplantation ↓ 12 days | 1 | 23.2, 21.1, 21.8, 17.7, 20.5 | 20.0±0.90 | 445, 500, 665, 486, 650 | 549.0±45.2 |
| | 2 | 23.5, 21.1, 22.5, 17.5, 21.7 | 21.4±1.03 | 650, 600, 936, 650, 1008 | 768.8±84.2 |
| | 3 | 24.3, 23.3, 23.3, 16.5, 22.5 | 21.9±1.39 | 847, 600, 1267, 1008, 1568 | 1058.0±167.4 |
| | 4 | 24.0, 22.9, 22.2, d, 22.0 | 22.7±0.45 | 1000, 936, 2025, d, 1912 | 1488.2±279.7 |
| | 5 | 22.4, 22.9, 22.6, d, 21.9 | 22.7±0.31 | 1352, 936, 2250, d, 2432 | 1742.57±357.7 |
| | 6 | 25.4, 22.6, 24.0, d, 23.6 | 23.9±0.58 | 2432, 1568, 2816, d, 3240 | 2514.0±355.8 |
| | 7 | 25.5, 23.4, 24.3, d, 23.6 | 24.2±0.47 | 3402, 3564, 4512, d, 4400 | 3969.5±283.7 |
| | 8 | 24.4, 22.9, 24.3, d, 23.3 | 23.7±0.37 | 3917, 4151, 5200, d, 4800 | 4530.53±285.4 |
| | 12 | 25.4, 22.9, 25.2, d, 23.4 | 24.2±0.63 | 5000, 5008, 5953, d, 5953 | 5678.5±228.7 |
| | 15 | 27.4, 23.8, 28.5, d, 23.8 | 25.8±1.21 | 8750, 7776, 10080, d, 10816 | 9355.58±678.1 |
| | 19 | 29.6, 21.0, 31.4, d, 23.9 | 27.2±1.92 | 14400, 12028, 15288, d, 11154 | 13127.5±792.8 |
| | 22 | 32.2, 26.6, 27.8, d, 25.4 | 28.0±1.48 | 15300, 16200, 18000, d, 12028 | 15382.0±1250.9 |
| | 25 | 32.6, 27.8, 20.5, d, 26.4 | 28.8±1.33 | 17298, 18944, 20661, d, 15750 | 18162.0±1057.8 |

Table 14 (Continued)

Example 37 Compound M (200 mg/kg)

| Number of days after transplantation of tumor | | Body weight (g) Range | Body weight (g) Average value±SE | Volume of tumor (mm3) Range | Volume of tumor (mm3) Average value±SE | T/C (%) |
|---|---|---|---|---|---|---|
| Tumor transplantation ↓ | | | | | | |
| 12 days | 1 | 22.7, 20.4, 20.3, 21.3, 19.6 | 20.8±0.53 | 288, 405, 650, 500, 455 | 457.6±59.3 | - |
| | 2 | 24.0, 22.0, 21.1, 22.5, 21.9 | 22.3±0.48 | 144#,455, 567, 550, 455 | 501.7±32.9 | 65.2* |
| | 3 | 24.3, 21.4, 21.1, 22.2, 22.1 | 22.2±0.55 | 256, 405, 1224, 550, 650 | 617.0±165.7 | 58.3 |
| | 4 | 23.9, 19.5, 20.9, 21.5, 22.0 | 21.5±0.71 | 256,500, 968, 600, 726 | 610.0±118.1 | 40.9** |
| | 5 | 23.3, 18.7, 20.5, 20.7, 21.4 | 20.9±0.74 | 405, 550, 1028, 726, 847 | 711.2±109.2 | 40.8** |
| | 6 | 24.5, 20.4, 21.5, 21.9, 22.4 | 22.1±0.67 | 550, 600, 1521, 1080, 1267 | 1003.6±188.6 | 39.9** |
| | 7 | 23.8, 20.6, 20.3, 22.1, 23.4 | 22.0±0.70 | 726, 786, 1224, 1080, 1352 | 1033.6±121.5 | 26.0** |
| | 8 | 21.4, 20.2, 19.5, 21.5, 22.4 | 21.0±0.51 | 1080, 786, 1605, 1152, 1666 | 1257.8±166.2 | 27.7** |
| | 12 | 23.2, 20.4, 21.2, 22.0, 23.0 | 21.9±0.53 | 3564, 1470, 2475, 1764, 2560 | 2366.6±364.0 | 41.6** |
| | 15 | 26.3, 21.0, 22.8, 22.9, 24.1 | 23.4±0.87 | 5733, 2916, 5405, 3402, 5000 | 4493.2±562.2 | 48.0** |
| | 19 | 28.3, 19.5, 26.2, 25.0, 24.2 | 24.6±1.45 | 9375, 3240, 6400, 4800, 4860 | 5735.0±1038.1 | 43.3** |
| | 22 | 30.7, 22.4, 27.6, 25.7, 25.6 | 26.3±1.35 | 13328, 5200, 8750, 7141, 7744 | 8493.2±1352.2 | 54.8** |
| | 25 | 31.0, 23.4, 28.5, 26.0, 26.2 | 27.0±1.28 | 15750, 5953, 9062, 8064, 9504 | 9672.6±1638.0 | 53.2** |

#: $P<0.05$ (delete)

*: $P<0.05$, **$P<0.02$, ***$P<0.01$, ****$P<0.001$

Examples 38 and 39 and Comparative Example 29

A patch of human lung cancer [cancer cells recommended by National Cancer Institute (NCI) of the U.S.A.] (OAT) measuring approximately 1 x 1 mm in area was transplanted beneath the hepatic capsule of each of female ICR/cd-1 mice (18 to 20 g) five to six weeks old (SRC method).

Then, an aqueous solution of 1% by weight of the aforementioned compound M was administered by intraperitoneal injection (ip) at a dose of 100 mg/kg and a dose of 200 mg/kg to the mice once per day for 5 consecutive days. The body weights of the mice and the sizes of tumor in the mice were measured on the 7th day, i.e. after one days interval following the stop of the administration to evaluate the effect of compound. The results were as shown in Table 15.

## Table 15

| Example | Active Compound | Dosage (mg/kg) | Treatment Schedule (days) | Body weight: W0 | Body weight: W7 | Body weight: W7/W0 | Tumorous activity: T0(omu) | Tumorous activity: T7(omu) | Tumorous activity: T7/T0 | T/C (%) | Pr |
|---|---|---|---|---|---|---|---|---|---|---|---|
| Comparative Example 29 | Control (no drug) | - | - | 20.0 | 23.0 | 1.15 | 10.0 | 27.5 | 2.75 | | - |
| | | | | 21.1 | 24.0 | 1.08 | 10.0 | 26.0 | 2.60 | - | |
| | | | | 21.3 | 23.2 | 1.09 | 10.0 | 24.0 | 2.40 | | |
| | | | | | | 1.11±0.02 | | | 2.58±0.10 | | |
| Example 38 | Compound M | 100 | IP 1~6 (except for 4) | 18.9 | 22.5 | 1.19 | 10.0 | 22.5 | 2.25 | | |
| | | | | 19.4 | 22.0 | 1.13 | 10.0 | 16.0 | 1.60 | | |
| | | | | 20.7 | 23.2 | 1.12 | 10.0 | 21.5 | 2.15 | 77.4 | |
| | | | | | | 1.14±0.02 | | | 2.00±0.20 | | |
| Example 39 | Compound M | 100 | IP 1~6 | 21.2 | 23.2 | 1.09 | 10.0 | 12.5 | 1.25 | | |
| | | | | 19.4 | 23.7 | 1.22 | 10.0 | 14.0 | 1.40 | | |
| | | | | 19.5 | 21.2 | 1.09 | 10.0 | 20.5 | 2.05 | 60.0 | 0.02 |
| | | | | | | 1.13±0.04 | | | 1.56±0.24 | | |

W0 = Body weight of mouse on 0 day.
W7 = Body weight of mouse on 7th day.
To = Size of tumor on 0 day (Major diameter + minor diametr)/2
T7 = Size of tumor on 7th day (Major diamter + minor diameter)/2
A patch of tumor (1 $mm^2$) was transplanted under the nepatic capsule of the female ICR mouse under anesthesis. On elapse of 24 hours following the transplantation. The compound M was intraperitoneally administered once daily on the first through 6th days except for the 4th day.
The final measurement of the sizes of tumor was made 7 days after the transplantation

It is clearly noted from Table 15 that the compound M according with this invention showed strong antineoplastic activity.

Examples 40 and 41 and Comparative Example 30

(Evaluation by size of tumor)

The heteropoly acid salt (Compound M) mentioned above was dissolved in distilled water to obtain an aqueous solution containing the compound in a concentration of 10 mg/ml. This aqueous solution was administered to female C3H/He mice (five weeks old) to which MM-46 breast cancer had been transplanted, to test the compound for antineoplastic activity. To be more specific, the MM-46 breast cancer was transplanted to the mice in a ratio of 1.0 x $10^5$ cells per mouse and the aqueous solution was administered (at a dose of 100 mg and a dose of 200 mg per kg of body weight) intraperitoneally (ip) or orally (po) for 9 consecutive days. After elapse of 14 days following the stop of the administration, the sizes of tumor were measured. The results were as shown in Table 16. For comparison, mice to which no aqueous solution was administered were similarly tested. The results were as shown in Table 16.

Table 16

| Example | Active substance | Dosage (mg/kg/day) | Treatment schedule | Change in body weight 14 days (g) | Range of tumor weight 21 days after transplantation (mg) | Average weight of tumor 21 days after transplantation (mg) ±SE | T/C | Number of tumorless mice/total number of mice |
|---|---|---|---|---|---|---|---|---|
| Comparative Example 30 | Control (no drug) | - | - | (+)6.39 | 1952, 3104, 1438, 3164, 1463, 1995, 3186, 885, 981 | 2024±308(9) | - | 0/9 |
| Example 40 | Compound M | 200 po×9 | D1-D9 | (+)4.70 | 512, 425, 400, 799, 310, 683, 1546 | 572±76(6)*** | 0.258 | 0/7 |
| Example 41 | Compound M | 100 ip×9 | D1-D9 | (+)4.36 | 520, 84, 42, 766, 31, 1219 | 560±283(6)*** | 0.277 | 0/6 |

- Significant error from the value obtained of Control group of tumor, *** $p<0.01$, **** $p<0.001$, Deletion ($Pr<0.05$)
- To the female FC3H/He mice, the MM-46 breast cancer was subcutaneously transplanted in a ratio of $1.0\times10^5$ cells per mouse on 0 day, and the compound M was orally or intraperitoneally administered at the prescribed dose during the period of preservation, i.e. 9 consecutive days on elapse of 24 hours following the transplantation of tumor.
- The measurement of the sizes of tumor in the mice was made 21 days after the transplantation of tumor.

It is clearly noted from the results of test given above that the compound according with the present invention showed an effect in suppressing the hyperplasia of tumor in all of the cases involving intraperitoneal and oral administration. As regards toxicity, the compound showed very slight toxicity as evinced by a satisfaotory increase of body weight attained even after 9 consecutive days' administration at a dose of 100 mg/kg. ip and 200 mg/kg po. Other compounds according with this invention, but not shown in the table, showed a similar effect in suppressing the hyperplasia of tumor.

Examples 42 and 43 and Comparative Example 31

(Evaluation by elongation of period of survival)

The aforementioned active compound M was dissolved in distilled water to obtain an aqueous solution containing the compound in a concentration of 10 mg/ml. To female C3H/He mice (five weeks old) to which MM-46 breast cancer was transplanted in a ratio of 1.0 x $10^5$ cells per mouse, the aqueous solution was administered intraperitoneally (ip) or orally (po) at a dose of 100 mg and a dose of 200 mg per kg of body weight for 9 consecutive days to measure the periods of the mices survival. The results were as shown in Table 17. For comparison, mice to which no aqueous solution was administered was similarly tested. The results were as shown in Table 17.

Table 17

| Example | Active substance | Dosage (mg/kg/day) | Treatment schedule | Change in body weight 14 days (g) | Range of number of days of survival (days) | Average days of survival (days) ±SE | % ILS | |
|---|---|---|---|---|---|---|---|---|
| Comparative Example 31 | Control (no drug) | - | - | (+)6.39 | 21, 22, 25, 29, 32, 32, 33, 21, 32 | 27.4±1.7(9) | - | 0/9 |
| Example 42 | Compound M | 200 po×9 | D1-D9 | (+)4.70 | 41, 33, 39, 60, 41, 49 | 43.8±3.9(6)**** | 59.7 | 0/6 |
| Example 43 | Compound M | 100 ip×9 | D1-D9 | (+)4.36 | 61, 90, 78, 28, 53, 43, 33 | 55.1±8.7(7)*** | 100.9 | 1/7 |

- Significance from the value obtained of Control group of tumor, * $p<0.05$, ** $p<0.02$, *** $p<0.01$, **** $p<0.001$
- To the female C3H/He mice, the MM46 breast cancer was subcutaneously transplanted in a ratio of $1.0\times10^5$ cells per mouse on 0 day, and the compound M was orally or intraperitoneally administered during the period of preservation, i.e. 9 consecutive days, on elaspe of 24 hours following the transplantation.

It is clearly noted from the results of test given above that the active compound according with the present invention (Compound M, for example) showed a significant effect of elongation of life as evinced by ratios of elongation of life, 100.9% in the case of intraperitoneal administration and 59.7% in the case of oral administration, in terms of period of survival. Other compounds according with the present invention, but not shown in the table, showed a similar effect in elongation of life.

Industrial Applicability

As described above, this invention is directed to antineoplastic drugs which have as principal components thereof polymolybdates or heteropoly acid salts represented by the general formulas I to III mentioned above. The antineoplastic drugs, therefore, possess high degrees of antineoplastic activity and manifest conspicuous effects on such solid cancers as breast cancer, lung cancer, and sarcoma in particular. They exhibit distinctly high levels of antineoplastic activity as compared with aminonitrosourea which has been in popular use to date and show very low toxicity. Since they can be converted into aqueous solutions, they permit very easy handling and allow stable formulation.

## Claims

1. An antineoplastic drug having as an active component thereof a polymolybdate represented by the following general formula I:
$[A]_m[Mo_xO_yH_z]_n \bullet \ell H_2O$
wherein A stands for

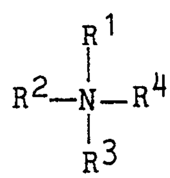

wherein $R^1$, $R^2$, $R^3$, and $R^4$ independently stand for hydrogen atom or a lower alkyl group, which may be identical or not, an alkali metal atom, or mixture of an alkali metal atom with other alkali metal atom, hydrogen atom, an ammonium group, or an alkyl ammonium group, providing that the number of carbon atoms of the alkyl group is an integer in the range of 1 to 3, m for an integer in the range of 1 to 16, n for an integer such that the negative charge, xn, assumes the value -m, x for an integer in the range of 2 to 36, y for an integer in the range of 4 to 112, z for an integer in the range of 0 to 24, and l for an integer in the range of 0 to 80].

2. An antineoplastic drug according to claim 1, wherein A in said general formula stands for

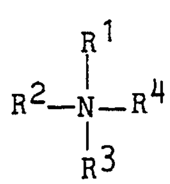

wherein $R^1$, $R^2$, $R^3_7$ and $R^4$ independently stand for a hydrogen atom or a lower alkyl group.

3. An antineoplastic drug according to claim 2, wherein said polymolybdate is in the form of an aqueous solution.

4. An antineoplastic drug according to claim 3, wherein said aqueous solution is stable at a pH value in the range of 5 to 8.

5. An antineoplastic drug according to any one of claims 2 to 4, in General Formula I of which R' stands for hydrogen atom, and $R^2$, $R^3$, and $R^4$ independently stand for hydrogen atom or an alkyl group of 1 to 6 carbon atoms except for the case in which $R^2$ to $R^4$ invariably stand for a hydrogen atom, m stands for an integer in the range of 2 to 8, n for an integer in the range of 2 to 8, x for an integer in the range of 2 to 20, y for an integer in the range of 10 to 15, z for an integer in the range of 0 to 10, and $\ell$ for an integer in the range of 1 to 10.

6. An antineoplastic drug according to claim 5, wherein said polymolybdate is $[NH_3C_3H_7(iso)]_6[Mo_7O_{24}] \bullet 3H_2O$.

7. An antineoplastic drug according to claim 5, wherein said polymolybdate is $[NH_3C_3H_7(iso)]_4 [Mo_{13}O_{40}]_{1/3} \bullet [Mo_{12}O_{40}H_2]_{2/3} \bullet$

8† 8. An antineoplastic drug according to claim 5, wherein said polymolybdate is $[NH_3C_3H_7]_6[Mo_7O_{24}H]$.

9. An antineoplastic drug according to Claim 5, wherein said polymolybdate is $[NH_4]_6[Mo_7O_{24}] \bullet 4H_2O$.

10. An antineoplastic drug according to claim 1, in General Formula 1 of which A stands for an alkali metal atom or a mixture of an alkali metal atom with other alkali metal atom, hydrogen atom, an ammonium group, or an alkylammonium group, providing that the number of carbon atoms of said alkyl group is an integer in the range of 1 to 3.

11. An antineoplastic drug according to claim 10, wherein said polymolybdate is in the form of an aqueous solution.

12. An antineoplastic drug according to claim 11, wherein said aqueous solution is stable at a pH value in the range of 4 to 9.

13. An antineoplastic drug according to claim 10, wherein said polymolybdate is $A_4[Mo_7O_{24}] \bullet 4H_2O$ wherein A stands for an alkali metal atom or a mixture of an alkali metal atom with other alkali metal atom, hydrogen atom, an ammonium group, or an alkylammonium group, providing that the number of carbon atoms of said alkyl group is an integer in the range of 1 to 3.

14. An antineoplastic drug according to claim 10, wherein said polymolybdate is $K_6[Mo_7O_{24}] \bullet 4H_2O$.

15. An antineoplastic drug according to claim 10, wherein said polymolybdate is $Na_6[Mo_7O_{24}] \bullet 4H_2O$.

16. An antineoplastic drug according to claim 10, wherein said polymolybdate is $Li_6[Mo_7O_{24}] \bullet 4H_2O$ .

17. An antineoplastic drug according to olaim 10, wherein said polymolybdate is $Cs_6[Mo_7O_{24}] \bullet 4H_2O$.

18. An antineoplastic drug having as a principal component thereof a polymolybdate represented by the following general formula II:

$$B_p[Mo_uO_w(O_2)_t(OH)_v] \cdot KH_2O \qquad II$$

wherein B stands for an alkali metal atoms, ammonium group, alkyl ammonium groups, and a mixture thereof, p for an integer in the range of 2 to 10, u for an integer in the range of 3 to 10, w for an integer in the range of 1 to 30, t for an integer in the range of 1 to 15, v for an integer in the range of 0 to 5, and k for an integer in the range of 0 to 20.

19. An antineoplastic drug according to claim 18, wherein said polymolybdate is in the form of an aqueous solution.

20. An antineoplastic drug according to claim 19, wherein said aqueous solution is stable at a pH value in the range of in to 9.

**21.** An antineoplastic drug `according to claim 18, wherein said polymolybdate is $[B]_6[Mo_7O_{22}(O_2)_2] \bullet KH_2O$ wherein B stands for an alkali metal atoms, ammonium group, alkylammonium groups and mixture thereof, and K for an integer in the range of 0 to 20.

**22.** An antineoplastic drug according to Claim 21, wherein said polymolybdate is $K_6[Mo_7O_{22}(O_2)_2] \bullet 8H_2O$.

**23.** An antineoplastic drug according to claim 21, wherein said polymolybdate is $[NH_3(iso)]_6[Mo_7O_{22}(O_2)] \bullet 5H_2O$ wherein iso stands for an isopropyl group.

**24.** An antineoplastic drug according to claim 18, wherein said polymolybdate is $[B]_6[Mo_7O_{23}(O_2)] \bullet KH_2O$ wherein B stands for an alkali metal atom, ammonium group, alkylammonium groups and a mixture thereof, and k for an integer in the range of 0 to 20.

**25.** An antineoplastic drug according to claim 24, wherein said polymolybdate is $K_6[Mo_7O_{23}(O_2)] \bullet 8H_2O$

**26.** An antineoplastic drug according to claim 24, wherein said polymolybdate is $[NH_3(iso)]_6[Mo_7O_{23}(O_2)] \bullet 5H_2O$ wherein iso stands for an isopropyl group.

**27.** An antineoplastic drug having as a principal component thereof a heteropoly acid salt of molybdenum and tungsten represented by the following general formula III:

$$[D]_qH_r[XMo_sW_{6-s}O_{24}] \qquad (III)$$

wherein D stands for an alkali metal atom, an ammonium group, an alkylammonium group, providing that the number of carbon atoms of said alkyl group is an integer in the range of 1 to 5, or a mixture thereof, X for iodine, chromium, cobalt platinum, tellurium, nickel, manganese, aluminum, gallium, zinc, iron, or copper, q for an integer in the range of 0 to 16, r for an integer in the range of 0 to 16, and s for an integer in the range of 0 to 6.

**28.** An antineoplastic drug according to claim 27, wherein said polymolybdate is $Na_5[IMo_6O_{24}] \bullet 34H_2O$.

**29.** An antineoplastic drug `according to claim 27, wherein said polymolybdate is $[NH_4]_3H_4[CoMo_6O_{24}] \bullet 7H_2O$.

**30.** An antineoplastic drug according to claim 27, wherein said polymolybdate is $Na_3H_6[CrMo_6O_{24}] \bullet 8H_2O$.

FIG. I

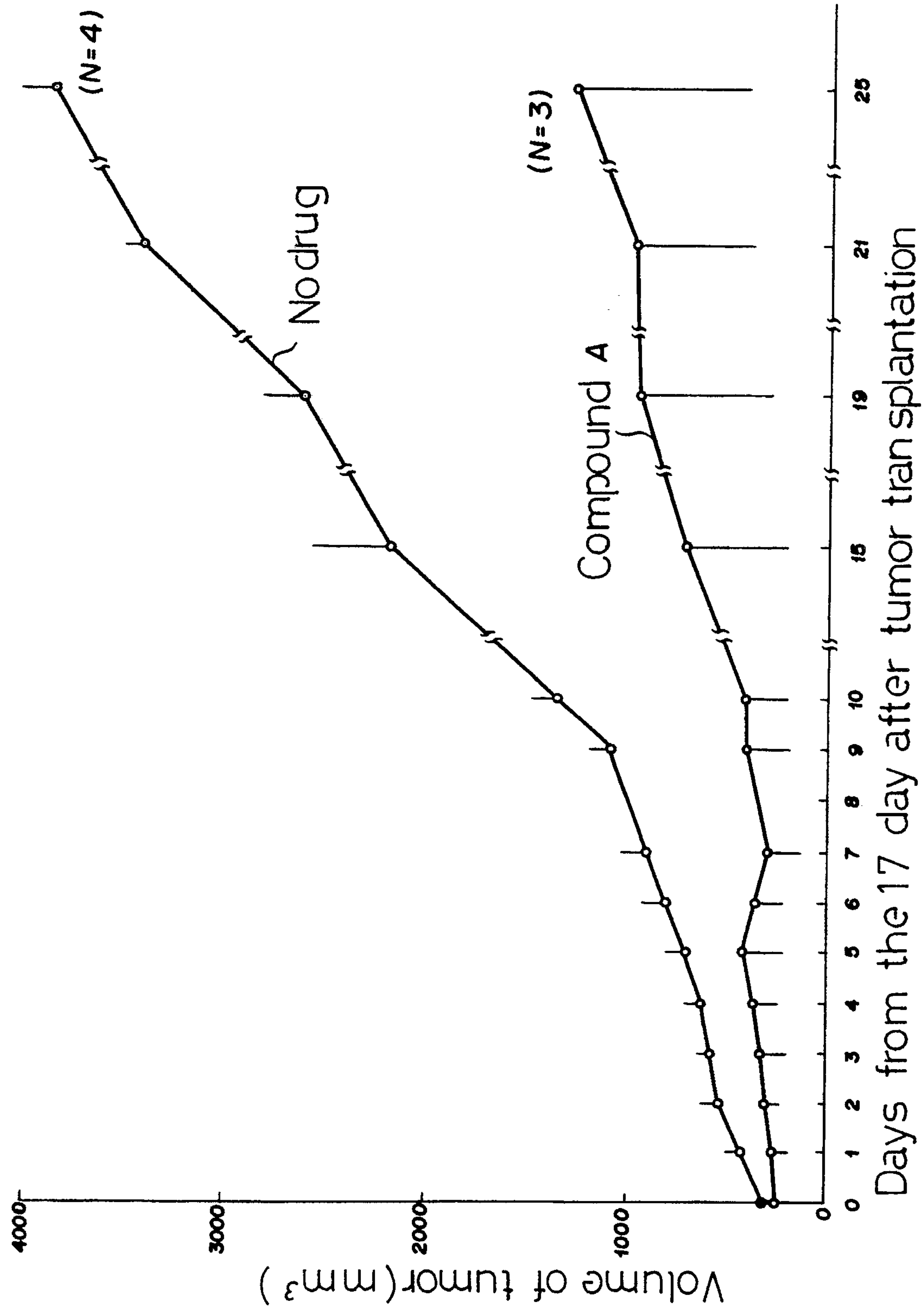

Volume of tumor (mm³)
4000
3000
2000
1000
0
Days from the 17 day after tumor transplantation
0
1
2
3
4
5
6
7
8
9
10
15
19
21
25
No drug
Compound A
(N=4)
(N=3)

FIG. 2

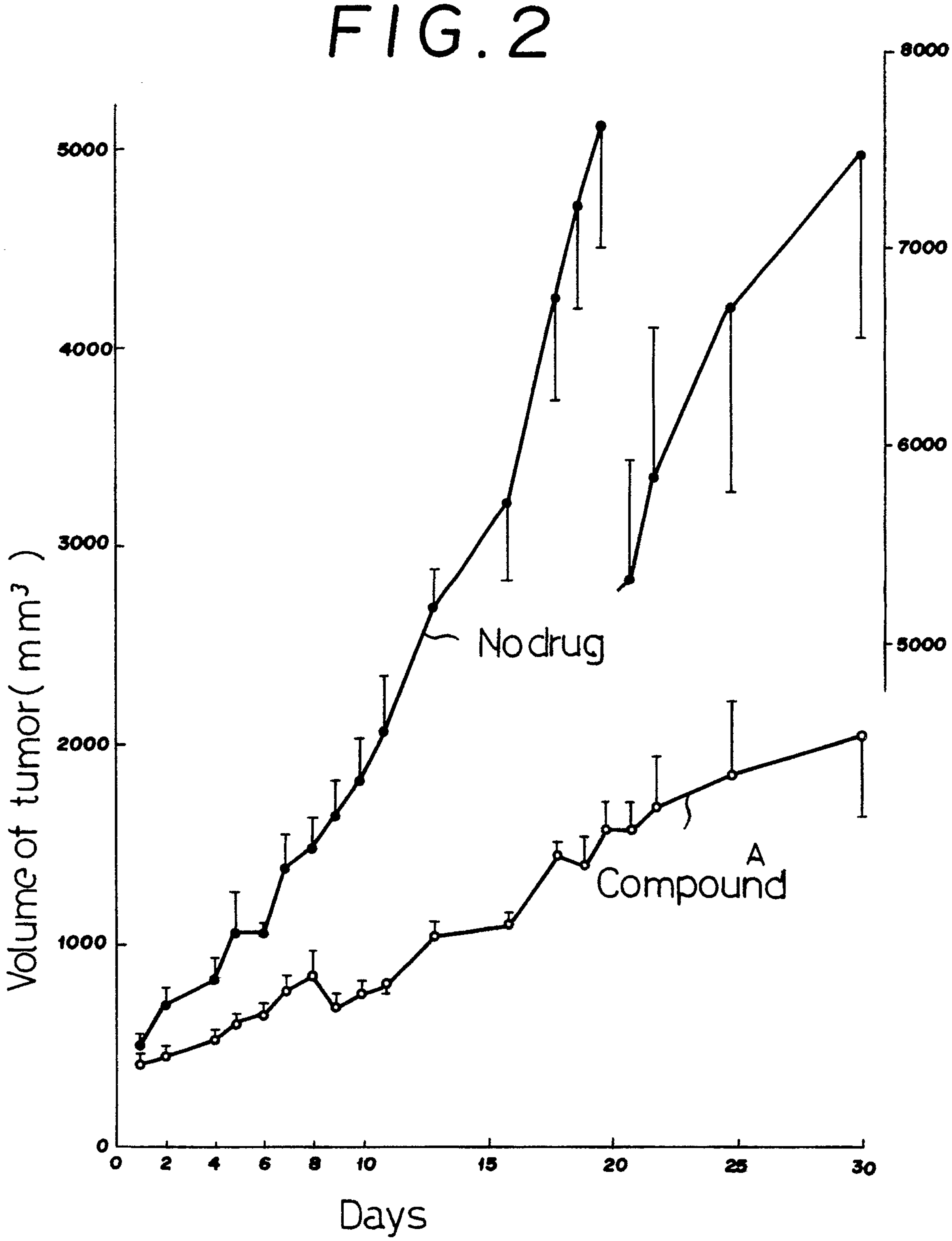

8000
7000
6000
5000
5000
4000
3000
2000
1000
0
Volume of tumor ( mm³ )
No drug
Compound A
0
2
4
6
8
10
15
20
25
30
Days

FIG. 3

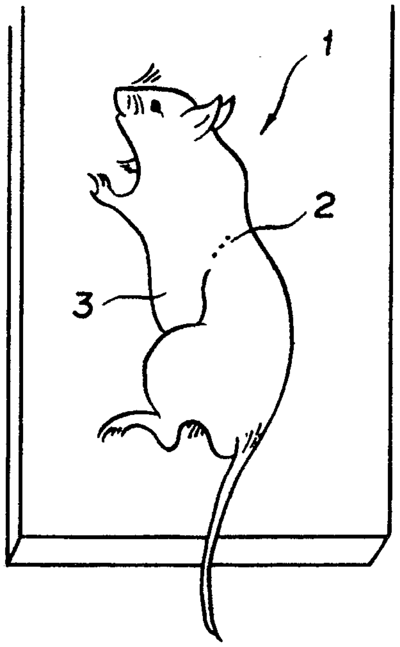


FIG. 4

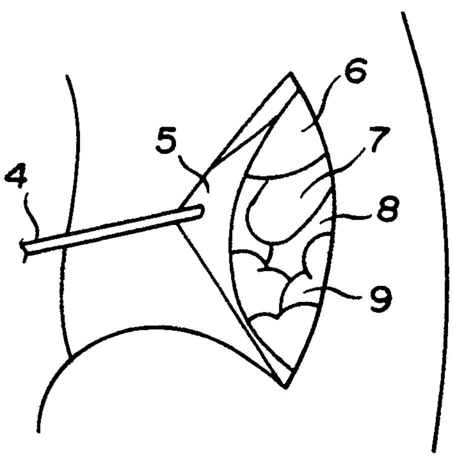


FIG. 5

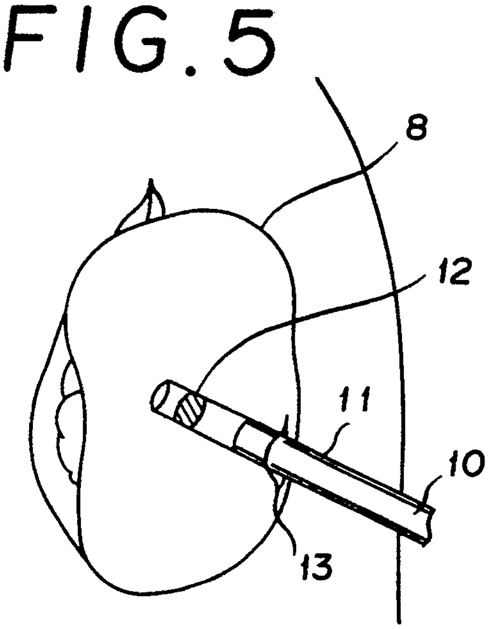

# INTERNATIONAL SEARCH REPORT

International Application No PCT/JP88/00559

**I. CLASSIFICATION OF SUBJECT MATTER** (if several classification symbols apply, indicate all)

According to International Patent Classification (IPC) or to both National Classification and IPC

Int.Cl$^4$ A61K31/28, A61K33/24

**II. FIELDS SEARCHED**

Minimum Documentation Searched

| Classification System | Classification Symbols |
|---|---|
| IPC | A61K31/28, A61K33/24 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| A | JP, A, 54-75192 (Toyo Seiyaku Kasei Kabushiki Kaisha) 15 June 1979 (15. 06. 79) Page 1 (Claim) (Family: none) | 1-30 |
| A | JP, A, 55-4348 (Joseph Pérez) 12 January 1980 (12. 01. 80) Page 1 (Claim 1), page 4, upper left column (Family: none) | 1-30 |
| A | GB, A, 1,385,489 (Agence Nationale de Valor) 26 February 1975 (26. 02. 75) & DE2,117,803 | 1-30 |
| A | JP, A, 61-63619 (Kasano Hiroyuki) 1 April 1986 (01. 04. 86) Page 1, lower left column (Family: none) | 1-30 |
| P | JP, A, 62-230619 (Polytoronics Kabushiki Kaisha) 9 October 1987 (09. 10. 87) | 1-30 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 29, 1988 (29. 08. 88) | September 12, 1988 (12. 09. 88) |
| **International Searching Authority** | **Signature of Authorized Officer** |
| Japanese Patent Office | |

International Application No PCT/JP88/00559

**FURTHER INFORMATION CONTINUED FROM THE SECOND SHEET**

Page 1, lower left column, page 4, upper right column, line 13 to page 4, lower right column, line 5 (Family: none)

| | | |
|---|---|---|
| P | JP, A, 62-230620 (Polytoronics Kabushiki Kaisha) 9 October 1987 (09. 10. 87) Page 1, lower left column, page 4, upper right column, 7th line from the bottom to page 4, lower left column, line 10 (Family: none) | 1-30 |

**V. ☐ OBSERVATIONS WHERE CERTAIN CLAIMS WERE FOUND UNSEARCHABLE**

This international search report has not been established in respect of certain claims under Article 17(2) (a) for the following reasons

1. ☐ Claim numbers............, because they relate to subject matter not required to be searched by this Authority namely

2. ☐ Claim numbers............, because they relate to parts of the international application that do not comply with the prescribed requirements to such an extent that no meaningful international search can be carried out specifically

**VI. ☐ OBSERVATIONS WHERE UNITY OF INVENTION IS LACKING**

This International Searching Authority found multiple inventions in this international application as follows

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims, it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees